# EUROPÄISCHE PATENTSCHRIFT

(11) **EP 1 151 112 B1**
(45) Veröffentlichungstag und Bekanntmachung des Hinweises auf die Patenterteilung: **20.01.2010**
(21) Anmeldenummer: 00912457.9
(22) Anmeldetag: 11.02.2000
(51) Int. Cl.: C12N 15/55, C12N 9/16

(54) **HITZEINDUZIERBARER PROMOTOR**
HEAT-INDUCIBLE PROMOTER
PROMOTEUR THERMO-INDUCTIBLE

(30) Priorität: 11.02.1999 CH 27999
(43) Veröffentlichungstag der Anmeldung: 07.11.2001
(73) Patentinhaber: RHEIN BIOTECH GESELLSCHAFT FÜR NEUE BIOTECHNOLOGISCHE PROZESSE UND PRODUKTE MBH, 40595 Düsseldorf (DE); Gellissen, Gerd, Prof. Dr., 42489 Wülfrath (DE)
(72) Erfinder: ROMANO, Ivano, D-5080 Laufenburg (CH); GELLISSEN, Gerd, D-42489 Wülfrath (DE); DE VIRGILIO, Claudio, D-4102 Binningen (CH)
(74) Vertreter: Kahlhöfer, Hermann
(86) Internationale Anmeldenummer: PCT/EP2000/001144
(87) Internationale Veröffentlichungsnummer: WO 2000/047749

(56) Entgegenhaltungen:
- US-A- 5 792 921
- BLAZQUEZ, MIGUEL A. ET AL: "Trehalose-6-P synthase is dispersable for growth on glucose but not for spore germination in Schizosaccharomyces pombe" J. BACTERIOL. (1994), 176(13), 3895-902 , XP000914935
- REINDERS, ANKE ET AL: "The thermophilic yeast Hansenula polymorpha does not require trehalose synthesis for growth at high temperatures but does for normal acquisition of thermotolerance" J. BACTERIOL. (1999), 181(15), 4665-4668 , XP000914945 -& ROMANO I.: "Hansenula polymorpha TPS1 gene" EMBL DATABASE : ACCESSION NUMBER AJ010725, 2. März 1999 (1999-03-02), XP002140874
- WOLSCHEK M.F. ET AL: "The filamentous fungus Aspergillus niger contains two "differentially regulated" trehalose-6-phosphate synthase-encoding genes, tpsA and tpsB" J.BIOL.CHEM., vol. 272, no. 5, 31 January 1997 (1997-01-31), pages 2729-2735,

### SEQUENZPROTOKOLL

<110> RheinBiotech Gesellschaft für neue biotechnologische Prozesse und Produkte mbH
<120> Hitzeinduzierbarer Promotor
<130> PCT1106-01966
<140>
   <141>
<150> CH 1999 0279/99
   <151> 1999-02-11
<160> 27
<170> PatentIn Ver. 2.1
<210> 1
   <211> 792
   <212> DNA
   <213> Hansenula polymorpha
<400> 1
   cttaaatacc acaataggaa aattatcaat aaagcttttc ggatttcatt acgttatatc 60
   gcaaaaaaat agtcgagctt tctgaaccgt tcgttaataa aaaaatagtt ttttcagatt 120
   tctatgtgag gcagtcacga tagaattcca tcgaactcgt cagcgccaaa tgtgaatgcg 180
   gctttcaaaa gctttgtcga atttgggatg ggaatccatg aatcgaagat gtcaaaatgg 240
   gggatcacaa aagtacactc acgaggaaaa tcaaaacctt ctcgtacctt taacacatac 300
   ggaaatgatc gatcgatttg agaagattcc tcaatgattt tcgtcatata taggtatctg 360
   aggtatttat ggaccgattc gtaataacat catatacatc gcgctttgtc cctgtcccag 420
   agatttcgat gaaaaaagcg aattttattc taatatttga agcatgccaa acatggggca 480
   gttgatttgt gtgagggtaa aatatcatga attgcaccca tcaaatgcag caagatattg 540
   accaatccta taatagaaaa cagacttacc acaaatagat tgtgatgacg atattatgaa 600
   tctccagatg aaaggctcga aagctatgaa gcctcttgaa acttttcatg gtgagataat 660
   attttcgaaa tttccacgaa cttctaaaac gcaattattg aatataaagg aaaaataata 720
   tttccatata gcaagcaaat caagctgcac tcctcatcct taaaactaat aaatcttacc 780
   catttgatac ca                                                     792
<210> 2
   <211> 15
   <212> DNA
   <213> Künstliche Sequenz
<220>
   <223> Beschreibung der künstlichen Sequenz: Consensus-Sequenz eines Heatschock-Elementes
<400> 2 ngaannnnnn ngaan 15
<210> 3
   <211> 15
   <212> DNA
   <213> Künstliche Sequenz
<220>
   <223> Beschreibung der künstlichen Sequenz: Besondere Ausführungsform des Heatschock-Elementes
<400> 3 ngaannbwmn ngaan 15
<210> 4
   <211> 15
   <212> DNA
   <213> Künstliche Sequenz
<220>
   <223> Beschreibung der künstlichen Sequenz: Nukleinsäuresequenz eines Heatschock-Elementes
<400> 4
   tgaagcctct tgaaa 15
<210> 5
   <211> 15
   <212> DNA
   <213> Künstliche Sequenz
<220>
   <223> Beschreibung der künstlichen Sequenz: Nukleinsäuresequenz eines Heatschock-Elementes
<400> 5
   tgaatataaa ggaaa 15
<210> 6
   <211> 1903
   <212> DNA
   <213> Hansenula polymorpha
<400> 6
   atggtcaaag gtaatgttat agtggtttca aatagaatcc cagtcactat taagaagact 60
   gaagatgatg aaaatggaaa atcaagatac gactatacaa tgtcatcagg cggattagtg 120
   acggcattac aagggctcaa aaatccattt cgatggtttg gatggcctgg gatgtctgtt 180
   gatagcgaac agggacgaca aactgtcgag cgggatttga aggaaaagtt caattgttat 240
   ccgatatggt taagtgacga aattgcagac ttacattata acggctttag caattctata 300
   ctttggccat tgttccacta tcacccaggg gagatgaatt ttgatgaaat tgcttgggcc 360
   gcttatttgg aagcaaataa actgttttgc caaacgatct taaaggagat aaaagacggg 420
   gacgttatct gggtacatga ttatcatctc atgttgttgc cttcactgct aagagaccaa 480
   cttaatagta aggggctacc gaatgtcaaa attggctttt tccttcatac tccttttcct 540
   tcaagcgaaa tatacaggat acttcctgta aggaaagaaa ttctcgaagg agtgcttagt 600
   tgtgatttga taggtttcca cacctatgat tatgtccgtc actttcttag ttcggttgaa 660
   agaatattga aattgcgaac gagcccacaa ggtgttgtct ataatgatag acaggtgact 720
   gtaagtgctt atccgattgg cattgacgtt gacaaattct tgaatggtct taagactgat 780
   gaggtcaaaa gcaggataaa acagctggaa accagatttg gtaaagattg taaacttatt 840
   attggggtgg acaggctgga ttacatcaaa ggtgtacctc aaaaactcca cgcgtttgaa 900
   attttcttgg agagacaccc tgagtggatt ggaaaagttg ttttgataca ggtggctgtc 960
   ccctcacgag gggacgttga agaatatcaa tctttgaggg cagctgtaaa tgagctagtg 1020
   ggaagaatca atggtagatt tggtaccgtc gaatttgttc ctatccattt ccttcataaa 1080
   agcgtgaact tccaagagct gatatctgtc tacgctgcta gtgatgtttg tgtagtgtca 1140
   tcgacacggg acggaatgaa tttggtcagt tatgaataca ttgcttgtca acaagatcga 1200
   aagggatctc tagtactaag tgaatttgcg ggagctgctc agtcattaaa tggcgctctc 1260
   gtagtgaatc catggaatac agaagaactc agtgaagcta tttacgaagg cttgatcatg 1320
   agtgaagaga aaaggagggg caattttcag aagatgttca agtacattga gaaatatact 1380
   gcaagttatt ggggagagaa ctttgtgaaa gaattgacga gagtgtgatt actgtggttt 1440
   gcaggttaat ttgaaatgtt cacttgtact tgaagaattt tatattatat acatgttata 1500
   catcaatagg ataaaaatta agtagacaaa gttatcattt tgttgggctg taaaaattga 1560
   acgataacaa tatatttgac aaaattaatt tgatctaatt gagctggagg gcgtaatata 1620
   tttggtttcc tgaatcatct tgtagatcac aatatggggc agcttctttc gcagccgatc 1680
   acagagaaac acatcacact tgtccaacat gatcacatat cgcattcaat cggggaaatg 1740
   caaggataca ggttgaccat ggaagacgcg ttctgtgatt tgaacgaaag aatattcgtg 1800
   acggaagagg gacttgacat cagaaaacaa gacgagaata cagagggtga tctggagtct 1860
   cttcaaatta acatttatgg tgtctttgac ggacatggcg gtt 1903
<210> 7
   <211> 475
   <212> PRT
   <213> Hansenula polymorpha
<400> 7
   Met Val Lys Gly Asn Val Ile Val Val Ser Asn Arg Ile Pro Val Thr
   1               5                   10                  15
   Ile Lys Lys Thr Glu Asp Asp Glu Asn Gly Lys Ser Arg Tyr Asp Tyr
               20                  25                  30
   Thr Met Ser Ser Gly Gly Leu Val Thr Ala Leu Gln Gly Leu Lys Asn
           35                  40                  45
   Pro Phe Arg Trp Phe Gly Trp Pro Gly Met Ser Val Asp Ser Glu Gln
       50                  55                  60
   Gly Arg Gln Thr Val Glu Arg Asp Leu Lys Glu Lys Phe Asn Cys Tyr
   65                  70                  75                  80
   Pro Ile Trp Leu Ser Asp Glu Ile Ala Asp Leu His Tyr Asn Gly Phe
                   85                  90                  95
   Ser Asn Ser Ile Leu Trp Pro Leu Phe His Tyr His Pro Gly Glu Met
               100                 105                 110
   Asn Phe Asp Glu Ile Ala Trp Ala Ala Tyr Leu Glu Ala Asn Lys Leu
           115                 120                 125
   Phe Cys Gln Thr Ile Leu Lys Glu Ile Lys Asp Gly Asp Val Ile Trp
       130                 135                 140
   Val His Asp Tyr His Leu Met Leu Leu Pro Ser Leu Leu Arg Asp Gln
   145                 150                 155                 160
   Leu Asn Ser Lys Gly Leu Pro Asn Val Lys Ile Gly Phe Phe Leu His
                   165                 170                 175
   Thr Pro Phe Pro Ser Ser Glu Ile Tyr Arg Ile Leu Pro Val Arg Lys
               180                 185                 190
   Glu Ile Leu Glu Gly Val Leu Ser Cys Asp Leu Ile Gly Phe His Thr
           195                 200                 205
   Tyr Asp Tyr Val Arg His Phe Leu Ser Ser Val Glu Arg Ile Leu Lys
       210                 215                 220
   Leu Arg Thr Ser Pro Gln Gly Val Val Tyr Asn Asp Arg Gln Val Thr
   225                 230                 235                 240
   Val Ser Ala Tyr Pro Ile Gly Ile Asp Val Asp Lys Phe Leu Asn Gly
                   245                 250                 255
   Leu Lys Thr Asp Glu Val Lys Ser Arg Ile Lys Gln Leu Glu Thr Arg
               260                 265                 270
   Phe Gly Lys Asp Cys Lys Leu Ile Ile Gly Val Asp Arg Leu Asp Tyr
           275                 280                 285
   Ile Lys Gly Val Pro Gln Lys Leu His Ala Phe Glu Ile Phe Leu Glu
       290                 295                 300
   Arg His Pro Glu Trp Ile Gly Lys Val Val Leu Ile Gln Val Ala Val
   305                 310                 315                 320
   Pro Ser Arg Gly Asp Val Glu Glu Tyr Gln Ser Leu Arg Ala Ala Val
                   325                 330                 335
   Asn Glu Leu Val Gly Arg Ile Asn Gly Arg Phe Gly Thr Val Glu Phe
               340                 345                 350
   Val Pro Ile His Phe Leu His Lys Ser Val Asn Phe Gln Glu Leu Ile
           355                 360                 365
   Ser Val Tyr Ala Ala Ser Asp Val Cys Val Val Ser Ser Thr Arg Asp
       370                 375                 380
   Gly Met Asn Leu Val Ser Tyr Glu Tyr Ile Ala Cys Gln Gln Asp Arg
   385                 390                 395                 400
   Lys Gly Ser Leu Val Leu Ser Glu Phe Ala Gly Ala Ala Gln Ser Leu
                   405                 410                 415
   Asn Gly Ala Leu Val Val Asn Pro Trp Asn Thr Glu Glu Leu Ser Glu
               420                 425                 430
   Ala Ile Tyr Glu Gly Leu Ile Met Ser Glu Glu Lys Arg Arg Gly Asn
           435                 440                 445
   Phe Gln Lys Met Phe Lys Tyr Ile Glu Lys Tyr Thr Ala Ser Tyr Trp
       450                 455                 460
   Gly Glu Asn Phe Val Lys Glu Leu Thr Arg Val
   465                 470                 475
<210> 8
   <211> 2695
   <212> DNA
   <213> Hansenula polymorpha
<400> 8
   cttaaatacc acaataggaa aattatcaat aaagcttttc ggatttcatt acgttatatc 60
   gcaaaaaaat agtcgagctt tctgaaccgt tcgttaataa aaaaatagtt ttttcagatt 120
   tctatgtgag gcagtcacga tagaattcca tcgaactcgt cagcgccaaa tgtgaatgcg 180
   gctttcaaaa gctttgtcga atttgggatg ggaatccatg aatcgaagat gtcaaaatgg 240
   gggatcacaa aagtacactc acgaggaaaa tcaaaacctt ctcgtacctt taacacatac 300
   ggaaatgatc gatcgatttg agaagattcc tcaatgattt tcgtcatata taggtatctg 360
   aggtatttat ggaccgattc gtaataacat catatacatc gcgctttgtc cctgtcccag 420
   agatttcgat gaaaaaagcg aattttattc taatatttga agcatgccaa acatggggca 480
   gttgatttgt gtgagggtaa aatatcatga attgcaccca tcaaatgcag caagatattg 540
   accaatccta taatagaaaa cagacttacc acaaatagat tgtgatgacg atattatgaa 600
   tctccagatg aaaggctcga aagctatgaa gcctcttgaa acttttcatg gtgagataat 660
   attttcgaaa tttccacgaa cttctaaaac gcaattattg aatataaagg aaaaataata 720
   tttccatata gcaagcaaat caagctgcac tcctcatcct taaaactaat aaatcttacc 780
   catttgatac caatggtcaa aggtaatgtt atagtggttt caaatagaat cccagtcact 840
   attaagaaga ctgaagatga tgaaaatgga aaatcaagat acgactatac aatgtcatca 900
   ggcggattag tgacggcatt acaagggctc aaaaatccat ttcgatggtt tggatggcct 960
   gggatgtctg ttgatagcga acagggacga caaactgtcg agcgggattt gaaggaaaag 1020
   ttcaattgtt atccgatatg gttaagtgac gaaattgcag acttacatta taacggcttt 1080
   agcaattcta tactttggcc attgttccac tatcacccag gggagatgaa ttttgatgaa 1140
   attgcttggg ccgcttattt ggaagcaaat aaactgtttt gccaaacgat cttaaaggag 1200
   ataaaagacg gggacgttat ctgggtacat gattatcatc tcatgttgtt gccttcactg 1260
   ctaagagacc aacttaatag taaggggcta ccgaatgtca aaattggctt tttccttcat 1320
   actccttttc cttcaagcga aatatacagg atacttcctg taaggaaaga aattctcgaa 1380
   ggagtgctta gttgtgattt gataggtttc cacacctatg attatgtccg tcactttctt 1440
   agttcggttg aaagaatatt gaaattgcga acgagcccac aaggtgttgt ctataatgat 1500
   agacaggtga ctgtaagtgc ttatccgatt ggcattgacg ttgacaaatt cttgaatggt 1560
   cttaagactg atgaggtcaa aagcaggata aaacagctgg aaaccagatt tggtaaagat 1620
   tgtaaactta ttattggggt ggacaggctg gattacatca aaggtgtacc tcaaaaactc 1680
   cacgcgtttg aaattttctt ggagagacac cctgagtgga ttggaaaagt tgttttgata 1740
   caggtggctg tcccctcacg aggggacgtt gaagaatatc aatctttgag ggcagctgta 1800
   aatgagctag tgggaagaat caatggtaga tttggtaccg tcgaatttgt tcctatccat 1860
   ttccttcata aaagcgtgaa cttccaagag ctgatatctg tctacgctgc tagtgatgtt 1920
   tgtgtagtgt catcgacacg ggacggaatg aatttggtca gttatgaata cattgcttgt 1980
   caacaagatc gaaagggatc tctagtacta agtgaatttg cgggagctgc tcagtcatta 2040
   aatggcgctc tcgtagtgaa tccatggaat acagaagaac tcagtgaagc tatttacgaa 2100
   ggcttgatca tgagtgaaga gaaaaggagg ggcaattttc agaagatgtt caagtacatt 2160
   gagaaatata ctgcaagtta ttggggagag aactttgtga aagaattgac gagagtgtga 2220
   ttactgtggt ttgcaggtta atttgaaatg ttcacttgta cttgaagaat tttatattat 2280
   atacatgtta tacatcaata ggataaaaat taagtagaca aagttatcat tttgttgggc 2340
   tgtaaaaatt gaacgataac aatatatttg acaaaattaa tttgatctaa ttgagctgga 2400
   gggcgtaata tatttggttt cctgaatcat cttgtagatc acaatatggg gcagcttctt 2460
   tcgcagccga tcacagagaa acacatcaca cttgtccaac atgatcacat atcgcattca 2520
   atcggggaaa tgcaaggata caggttgacc atggaagacg cgttctgtga tttgaacgaa 2580
   agaatattcg tgacggaaga gggacttgac atcagaaaac aagacgagaa tacagagggt 2640
   gatctggagt ctcttcaaat taacatttat ggtgtctttg acggacatgg cggtt      2695
<210> 9
   <211> 26
   <212> DNA
   <213> Künstliche Sequenz
<220>
   <223> Beschreibung der künstlichen Sequenz: PCR-Primer F1 (vorwärts)
<400> 9
   tggccvytnt tccaytacca tccygg 26
<210> 10
   <211> 24
   <212> DNA
   <213> Künstliche Sequenz
<220>
   <223> Beschreibung der künstlichen Sequenz: PCR-Primer R1 (rückwärts)
<400> 10
   ggcrtgbaay ttytghggha cacc 24
<210> 11
   <211> 23
   <212> DNA
   <213> Künstliche Sequenz
<220>
   <223> Beschreibung der künstlichen Sequenz: Sequenzierungsprimer F3 (vorwärts)
<400> 11
   ggaagcaaat aaactgtttt gcc 23
<210> 12
   <211> 23
   <212> DNA
   <213> Künstliche Sequenz
<220>
   <223> Beschreibung der künstlichen Sequenz: Sequenzierungsprimer F4 (vorwärts)
<400> 12
   ctgtaagtgc ttatccgatt ggc 23
<210> 13
   <211> 22
   <212> DNA
   <213> Künstliche Sequenz
<220>
   <223> Beschreibung der künstlichen Sequenz: Sequenzierungsprimer F6 (vorwärts)
<400> 13
   ggacgacaaa ctgtcgagcg gg 22
<210> 14
   <211> 22
   <212> DNA
   <213> Künstliche Sequenz
<220>
   <223> Beschreibung der künstlichen Sequenz: Sequenzierungsprimer F7 (vorwärts)
<400> 14
   catactcctt ttccttcaag cg 22
<210> 15
   <211> 21
   <212> DNA
   <213> Künstliche Sequenz
<220>
   <223> Beschreibung der künstlichen Sequenz: Sequenzierungsprimer F8 (vorwärts)
<400> 15
   aaagcgtgaa cttccaagag c 21
<210> 16
   <211> 22
   <212> DNA
   <213> Künstliche Sequenz
<220>
   <223> Beschreibung der künstlichen Sequenz: Sequenzierungsprimer F9 (vorwärts)
<400> 16
   gcgtgtgatt actgtggttt gc 22
<210> 17
   <211> 26
   <212> DNA
   <213> Künstliche Sequenz
<220>
   <223> Beschreibung der künstlichen Sequenz: Sequenzierungsprimer F10 (vorwärts)
<400> 17
   ggtgagataa tattttcgaa atttcc 26
<210> 18
   <211> 27
   <212> DNA
   <213> Künstliche Sequenz
<220>
   <223> Beschreibung der künstlichen Sequenz: Sequenzierungsprimer F11 (vorwärts)
<400> 18
   cccatcaaat gcagcaagat attgacc 27
<210> 19
   <211> 21
   <212> DNA
   <213> Künstliche Sequenz
<220>
   <223> Beschreibung der künstlichen Sequenz: Sequenzierungsprimer R3 (rückwärts)
<400> 19
   ccattcaaga atttgtcaac g 21
<210> 20
   <211> 23
   <212> DNA
   <213> Künstliche Sequenz
<220>
   <223> Beschreibung der künstlichen Sequenz: Sequenzierungsprimer R4 (rückwärts)
<400> 20 catgagatga taatcatgta ccc 23
<210> 21
   <211> 23
   <212> DNA
   <213> Künstliche Sequenz
<220>
   <223> Beschreibung der künstlichen Sequenz: Sequenzierungsprimer R5 (rückwärts)
<400> 21
   caattttgac attcggtagc ccc 23
<210> 22
   <211> 22
   <212> DNA
   <213> Künstliche Sequenz
<220>
   <223> Beschreibung der künstlichen Sequenz: Sequenzierungsprimer R6 (rückwärts)
<400> 22
   gtaatgccgt cactaatccg cc 22
<210> 23
   <211> 23
   <212> DNA
   <213> Künstliche Sequenz
<220>
   <223> Beschreibung der künstlichen Sequenz: Sequenzierungsprimer R7 (rückwärts)
<400> 23
   gaacatcttc tgaaaattgc ccc 23
<210> 24
   <211> 21
   <212> DNA
   <213> Künstliche Sequenz
<220>
   <223> Beschreibung der künstlichen Sequenz: Sequenzierungsprimer R8 (rückwärts)
<400> 24
   ctagctcatt tacagctgcc c 21
<210> 25
   <211> 25
   <212> DNA
   <213> Künstliche Sequenz
<220>
   <223> Beschreibung der künstlichen Sequenz: Sequenzierungsprimer R9 (rückwärts)
<400> 25
   catagctttc gagcctttca tctgg 25
<210> 26
   <211> 24
   <212> DNA
   <213> Künstliche Sequenz
<220>
   <223> Beschreibung der künstlichen Sequenz: Sequenzierungsprimer Plasm. F (vorwärts)
<400> 26
   ggcgagcccg atcttcccca tcgg 24
<210> 27
   <211> 26
   <212> DNA
   <213> Künstliche Sequenz
<220>
   <223> Beschreibung der künstlichen Sequenz: Sequenzierungsprimer Plasm. R (rückwärts)
<400> 27
   ctgctcgctt cgctacttgg agccac 26

## Beschreibung

Die vorliegende Erfindung betrifft Nukleinsäure-Moleküle, die einen hitzeinduzierbaren Promotor umfassen, sowie Expressionsvektoren und Wirtszellen, die mindestens ein erfindungsgemäßes Nukleinsäure-Molekül enthalten. Ferner betrifft die vorliegende Erfindung Kits und Verfahren zum Herstellen von einem oder mehreren Proteinen unter Verwendung der erfindungsgemäßen Nukleinsäure-Moleküle sowie verschiedene Verwendungen derselben.

Mikroorganismen können auf unterschiedliche Streß-Situationen, wie beispielsweise Hitze- oder Kälteschock, Ethanol, Schwermetallionen, Sauerstoffentzug oder Nahrungs-, insbesondere Glukoseentzug, reagieren. Es ist bekannt, daß Hefen und andere Pilze während Phasen reduzierten Wachstums Trehalose akkumulieren. Das sind meist jene Entwicklungsstadien, die u.a. austrocknungs- und hitzetolerant sind, wie Sporen, Konidien, Sklerozien oder Zellen in der stationären Wachstumsphase. Es ist ebenfalls bereits bekannt, daß *Saccharomyces* cerevisiae-Zellen während eines einstündigen Hitzeschocks von 27°C auf 40°C Trehalose akkumulieren und daß diese Trehalose-Akkumulation mit einer erhöhten Thermotoleranz korreliert. Durch gezielte Mutationen konnte bewiesen werden, daß Trehalose tatsächlich einen notwendigen Faktor für die Thermotoleranz-Induktion darstellt.

In den Promotorregionen streßinduzierter Gene, wie den für die Trehalose-Synthese verantwortlichen Genen von *S. cerevisiae,* sind sogenannte HSEs (heat shock elements) und STREs (stress responsive elements) vorhanden. Diese Elemente scheinen eine streßinduzierte, u.a. hitzeschockinduzierte Aktivierung von Streßgenen zu vermitteln. Es wird heute allgemein angenommen, daß Phosphorylierung von Msn2p und Msn4p via den Ras/cAMP-Weg die Msn2p- und Msn4p-Transkriptionsfaktoren hemmt. Fällt diese Hemmung weg (z.B. unter Streßbedingungen), werden Msn2p und Msn4p aktiv. Beteiligung an der Antwort auf die Streßbedingungen wird STREs mit der Sequenz CCCCT zugeschrieben.

Pilze und insbesondere Hefen stellen aufgrund ihrer Fähigkeit, co- und posttranslationale Modifikationen auszuführen, die den humanen Modifikationen ähnlich sind, attraktive Systeme für die Produktion rekombinanter Proteine dar. Zur Herstellung rekombinanter Proteine wird häufig die kodierende Sequenz eines Gens, das ein Protein von Interesse kodiert, unter der Kontrolle eines geeigneten heterologen Promotors exprimiert. Als besonders vorteilhaft haben sich sog. induzierbare Promotoren erwiesen, die durch bestimmte Umweltbedingungen induziert werden können. Beispielsweise bieten die Promotoren von Genen, die Schlüsselenzyme im methylotrophen Stoffwechsel kodieren, wie z.B. der MOX(Methanoloxidase)- oder der FMD(Formiatdehydrogenase)-Promotor, vielseitig nutzbare Möglichkeiten für eine stark von der C-Quelle regulierte Expression von heterologen Genen.

Für molekularbiologische Arbeiten wurden Expressionsvektoren hergestellt, die einen hitzeinduzierbaren Promotor, beispielsweise den des hsp70-Gens aus *Drosophila,* be-inhalten. Die bisher für die Hitzeschock-Induktion in Pilzzellen und insbesondere in Hefen verwendeten Promotoren haben den Nachteil, daß sie nicht selektiv auf Hitzeschock reagieren, so daß der An- und Ausschaltmechanismus nicht ausreichend gut gesteuert werden kann, was insbesondere bei der Produktion von zellschädigenden Proteinen zu Problemen führen kann. Beispielsweise weist der *TPS1-*Promotor von *S. cerevisiae* mehrere als allgemeine Streß-Elemente, sog. STRE-Elemente, bekannte Sequenzen auf, nämlich CCCCT bzw. AGGGG, aber nur eine allenfalls als Hitzeschock-Element (HSE) wirkende Sequenz, nämlich GGAACAGAACAATCG. Ferner werden die heute bekannten Promotoren infolge ihrer breiten Streßreaktion durch einen Streßfaktor für viele Anwendungen in einem nicht befriedigenden Ausmass aktiviert.

US 5,792,921 offenbart eine Pflanze, welche mit mindestens einem Gen transformiert ist, welches eine Sequenz umfasst, die für eine Komponente der Trehalose-Synthase kodiert, wobei diese Sequenz in einem offenen Leseraster mit einem Pflanzen-Promotor verbunden ist. Ein erfindungsgemäßes Nukleinsäure-Molekül wird jedoch in US 5,792,921 nicht offenbart. Zwar offenbart US 5,792,921 Promotoren für die Trehalose-6P-Synthase, diese weisen jedoch CCCCT- bzw. AGGGG-Sequenzen auf.

Blazquez et al. offenbaren in J. Bacteriol. (1994), Band 176 (13), Seiten 3.895-3.902 eine Trehalose-6P-Synthase aus *S. pombe.* Beschrieben wird insbesondere die Bedeutung der Trehalose-6P-Synthase für das Wachstum von *S. pomb-*Zellen auf Glukose und für das Keimen von Sporen. Auch Blaszqhez et al. offenbaren nicht ein erfindungsgemäßes Nukleinsäure-Molekül und auch nicht dessen Verwendung als Promotor bei der Expression von Genen, welche unter der Kontrolle dieses Promotors stehen.

Wolschek et al. offenbaren in J. Biol. Chem. (1997), Band 272 (5), Seiten 2.729-2.735 offenbart zwei Gene in *A. niger,* welche für eine Trehalose-6P-Synthase kodieren. Zwar beschreibt Wolschek et al. ebenso wie US 5,792,921 Promotoren für die Trehalose-Synthase, diese weisen jedoch ebenfalls CCCCT- bzw. AGGGG-Sequenzen auf.

Aufgabe der Erfindung ist es daher, einen möglichst selektiv hitzeinduzierbaren Promotor bereitzustellen, insbesondere einen Promotor, der in Pilzen und vor allem in Hefen aktiv und für die Proteinexpression bei hohen Temperaturen geeignet ist.

Erfindungsgemäß wird diese Aufgabe von einem Nukleinsäure-Molekül gelöst, das einen hitzeinduzierbaren Promotor umfaßt ,kein STRE- Element mit den sequenz CCCCT oder AGGGG enthält , und aus folgenden Nukleinsäuren ausgewählt ist:
(a) einer Nukleinsäure mit der in SEQ ID NO:1 angegebenen Sequenz;
(b) einer Nukleinsäure mit einer Sequenz, die auf einer Länge von 300 bp mindestens 60% Identität mit der in (a) angegebenen Sequenz aufweist; wobei die Sequenzidentität mit einem Lücken-Wert von 50 und einem Lückenlängen-Wert von 3 bestimmt wird;
(c) einer Nukleinsäure, die mit dem Gegenstrang einer der in (a) oder (b) angegebenen Nukleinsäuren unter stringenten Bedingungen hybridisiert;
(d) einem Fragment einer der in (a) bis (c) angegebenen Nukleinsäuren, das die Funktion des hitzeinduzierbaren Promotors behält;
(e) einer Kombination mehrerer der in (a) bis (d) angegebenen Nukleinsäuren, wobei die Sequenzen der Nukleinsäuren gleich oder verschieden sein können;
   oder
   von einem Nukleinsäure-Molekül mit einer Sequenz, die zu der Sequenz einer der in (a) bis (e) angegebenen Nukleinsäuren komplementär ist.

Der Begriff "hitzeinduzierbarer Promotor", wie hier verwendet, bezeichnet eine Nukleinsäuresequenz, die bei einer Temperaturerhöhung im Kulturmedium von 25°C auf mindestens 37°C, vorzugsweise auf 47°C eine Zunahme der Transkription (RNA-Synthese) eines unter der transkriptionellen Kontrolle des Promotors stehenden Gens um mindestens 50% herbeiführt.

Das Merkmal "Sequenz, die mit dem Gegenstrang einer der in (a), oder (b) angegebenen Nukleinsäuren hybridisiert", weist auf eine Sequenz hin, die unter stringenten Bedingungen mit dem Gegenstrang einer Nukleinsäure mit den in (a), oder (b) angegebenen Merkmalen hybridisiert. Beispielsweise können die Hybridisierungen bei 68°C in 2 x SSC oder nach dem Protokoll des Dioxygenin-Labelling-Kits der Firma Boehringer (Mannheim) durchgeführt werden. Ein weiteres Beispiel für stringente Hybridisierungsbedingungen sind z.B. Inkubation bei 65°C über Nacht in 7% SDS, 1% BSA, 1 mM EDTA, 250 mM Natriumphosphatpuffer (pH 7,2) und nachfolgendes Waschen bei 65°C mit 2 x SSC; 0, 1 % SDS.

Der dem Fachmann bekannte Ausdruck "% Identität" bezeichnet den Grad der Verwandtschaft zwischen zwei oder mehr DNA-Molekülen bzw. zwei oder mehr Polypeptid-Molekülen, der durch die Übereinstimmung zwischen den Sequenzen bestimmt wird. Der Prozentsatz der "Identität" ergibt sich aus dem Prozentsatz identischer Bereiche in zwei oder mehr Sequenzen unter Berücksichtigung von Lücken oder anderen Sequenzbesonderheiten.

Die Identität miteinander verwandter DNA-Moleküle oder Polypeptide kann mit Hilfe bekannter Verfahren bestimmt werden. In der Regel werden spezielle Computerprogramme mit den besonderen Anforderungen Rechnung tragenden Algorithmen eingesetzt. Bevorzugte Verfahren zur Bestimmung der Identität erzeugen zunächst die größte Übereinstimmung zwischen den untersuchten Sequenzen. Computerprogramme zur Bestimmung der Identität zwischen zwei Sequenzen umfassen, sind jedoch nicht eingeschränkt auf, das GCG-Programmpaket, einschließlich GAP (Devereux, J., et al., Nucleic Acids Research 12 (12): 387 (1984); Genetics Computer Group University of Wisconsin, Madison, (WI)); BLASTP, BLASTN und FASTA (Altschul, S. et al., J. Molec Biol 215:403/410 (1990)). Das BLAST X Programm kann vom National Centre for Biotechnology Information (NCBI) und aus weiteren Quellen bezogen werden (BLAST Handbuch, Altschul S., *et al.,* NCB NLM NIH Bethesda MD 20894; Altschul, S., et al., J. Mol. 215:403/410 (1990)). Auch der bekannte Smith Waterman-Algorithmus kann zur Bestimmung von Identität verwendet werden.

**Parameter für den Sequenz-Vergleich umfassen die nachstehenden:**

| | |
|---|---|
| Algorithmus: | Needleman und Wunsch, J. Mol. Biol 48:443-453 (1970) |
| Vergleichsmatrix: | Übereinstimmung (matches) =+10, |
| | Nichtübereinstimmung (mismatch) = 0 |
| Lücken-Wert (Gap Penalty): | 50 |
| Lückenlängen-Wert (Gap Length Penalty): | 3 |

Das GAP-Programm ist auch zur Verwendung mit den vorstehenden Parametern geeignet. Die vorstehenden Parameter sind die Standardparameter (default parameters) für Nukleinsäuresequenz-Vergleiche.

Weitere beispielhafte Algorithmen, Lücken-Öffnungs-Werte (gap opening penalties), Lückenausdehnungs-Werte (gap extension penalties), Vergleichsmatrizen einschließlich der im Programm-Handbuch, Wisconsin-Paket, Version 9, September 1997, genannten können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird.

Überraschenderweise wurde nun gefunden, daß die erfindungsgemäßen Nukleinsäure-Moleküle und insbesondere der Promotor des Trehalose-6-Phosphat-Synthase *(TSP1)-*Gens von *Hansenula polymorpha,* mindestens unter den ersten ca. 300 bp stromaufwärts der kodierenden Sequenz keines der STRE-Elemente enthält, die in *S. cerevi*siae gefunden wurden und von denen man annimmt, daß sie für die Stressreaktion einschließlich der Hitzeschock-Induktion dieses Gens primär verantwortlich sind, und daß dieser Promotor gut und sehr selektiv auf Hitze reagiert.

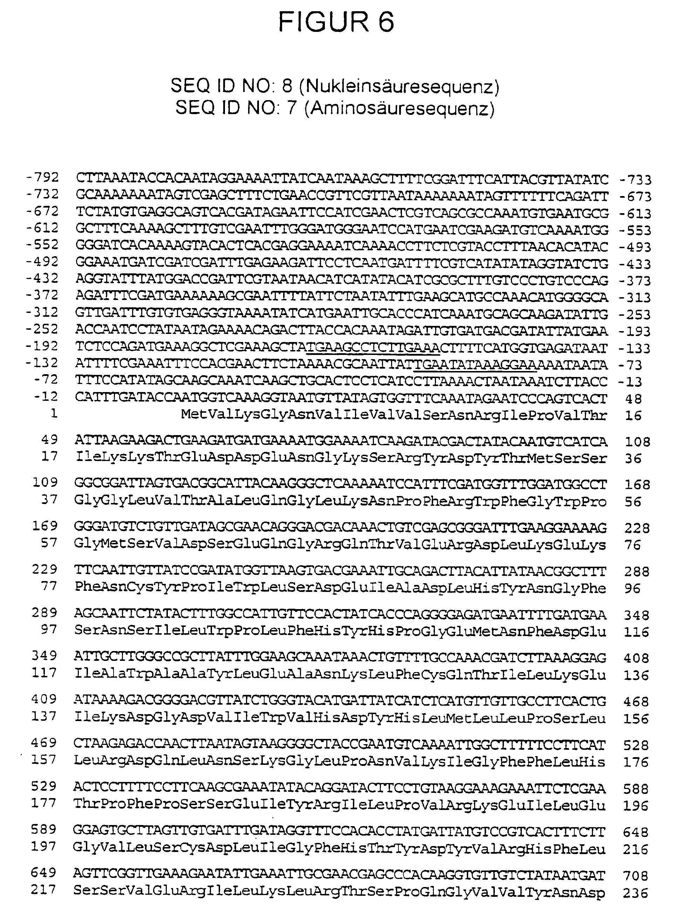

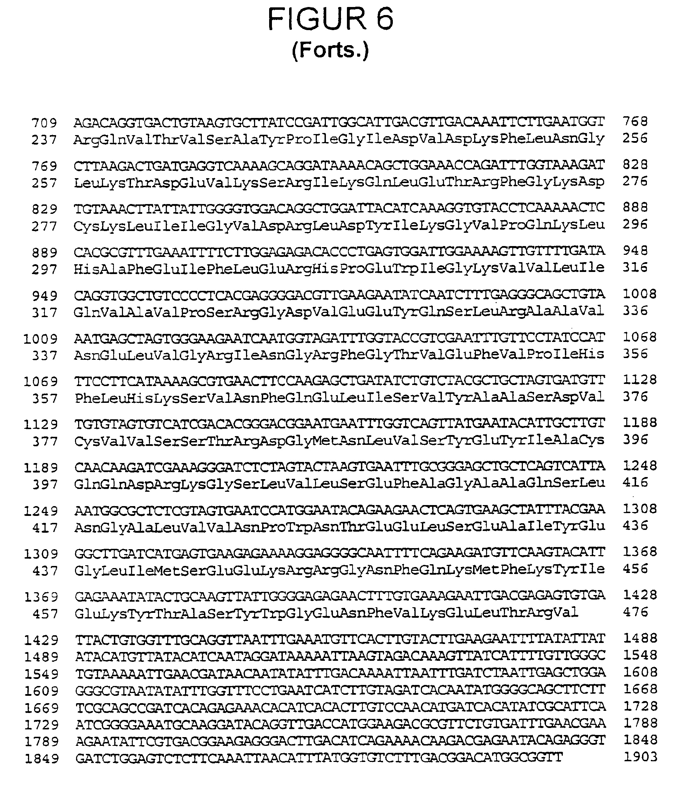

Die erfindungsgemäßen Nukleinsäure-Moleküle können entweder nach gängigen Verfahren synthetisch hergestellt, oder aber aus geeigneten DNA-Bibliotheken isoliert und gegebenenfalls anschliessend mutiert werden. Die Herstellung solcher Bibliotheken ist dem Fachmann ebenfalls bekannt. Eine Isolierung erfolgt vorzugsweise, indem eine Sonde mit einer Länge von mindestens 200 - 400 bp und der kodierenden Sequenz des *TPS1-*Gens von *H. polymorpha* (siehe Figur 6) hergestellt und damit eine DNA-Bibliothek, insbesondere eine genomische DNA-Bibliothek gescreent wird. Eine solche Sonde kann unter Verwendung von geeigneten Primern, welche jeweils vorzugsweise mindestens 20 - 21 bp lang sind und geeignete Sequenzen entsprechend Figur 6 (resp. der entsprechenden Komplementärsequenz) aufweisen, und genomischer oder cDNA von *H. polymorpha* als "Template", mittels PCR (Polymerase Chain Reaction) hergestellt werden.

Sonden können entweder synthetisiert oder aber durch Fragmentierung allenfalls vorhandener *TPS1-*DNA hergestellt werden.Selbstverständlich ist es auch möglich direkt mit Sonden entsprechend Teilen der Promotorsequenz zu screenen, ein solches Vorgehen ist aber infolge allenfalls mangelhafter Konservierung der Sequenz innerhalb nicht kodierender Teile weniger bevorzugt.

In einer Ausführungsform der erfindungsgemäßen Nukleinsäure-Moleküle weist die Sequenz der Nukleinsäure auf einer Länge von 300 bp mindestens 80% Identität mit der vorstehend unter (a) angegebenen Sequenzen auf.

Nukleinsäure-Moleküle, die einen hitzeinduzierbaren Promotor umfassen und auf einer Länge von 300 bp mindestens 90% Identität mit der vorstehend unter (a) angegebenen Sequenzen aufweisen, sind besonders bevorzugt. Am stärksten bevorzugt sind jedoch Nukleinsäure-Moleküle, die auf einer Länge von 300 bp mindestens 95% Identität mit der vorstehend unter (a) angegebenen Sequenzen aufweisen.

Zur Ausführung der Erfindung bevorzugte Nukleinsäure-Moleküle weisen mindestens ein Hitzeschock-Element mit der Sequenz NGAANNNNNNNGAAN (SEQ ID NO:2) oder deren Komplementärsequenz auf, wobei die mit N bezeichneten Nukleotide unabhängig voneinander A,T, C und G sein können. Vorzugsweise weisen die erfindungsgemäßen Nukleinsäure-Moleküle mindestens ein Hitzeschock-Element mit der Sequenz NGAANNBWMNNGAAN (SEQ ID NO:3) oder deren Komplementärsequenz auf, wobei B ein G, C oder T, W ein A oder T, und M ein C oder A ist.

In einer besonders bevorzugten Ausführungsform der Erfindung ist das Hitzeschock-Element aus TGAAGCCTCTTGAAA (SEQ ID NO:4) und/oder TGAATATAAAGGAAA (SEQ ID NO:5) und/oder deren Komplementärsequenzen ausgewählt, wobei wenn zwei oder mehrere Hitzeschock-Elemente vorhanden sind, diese gleiche oder verschiedene Sequenzen aufweisen können. Ein bevorzugtes erfindungsgemäßes Nukleinsäure-Molekül weist mindestens zwei verschiedene Hitzeschock-Elemente auf.
Die erfindungsgemäßen Nukleinsäure-Moleküle enthalten kein STRE-Element mit der Sequenz CCCCT oder AGGGG.

Von der Erfindung werden ebenfalls Fragmente der vorgenannten erfindungsgemäßen Nukleinsäure-Moleküle bereitgestellt, die die Funktion des hitzeinduzierbaren Promotors behalten. Besonders bevorzugt ist ein Fragment, das die Sequenz von Nukleotid 228 bis Nukleotid 792 in der SEQ ID NO:1 umfaßt. Ein weiteres bevorzugtes Fragment umfaßt die Sequenz von Nukleotid 493 bis Nukleotid 792 in der SEQ ID NO:1. Ein Fragment, das die Sequenz von Nukleotid 627 bis Nukleotid 713 in der SEQ ID NO:1 umfaßt, kann ebenfalls verwendet werden.

Die erfindungsgemäßen Nukleinsäure-Moleküle können weiterhin mindestens eine unter der Transkriptionskontrolle des hitzeinduzierbaren Promotors stehende Nukleinsäuresequenz für ein heterologes Gen umfassen.

Unter einem "heterologen Gen" ist der kodierende Bereich eines Strukturgens zu verstehen, der entweder nicht unter der Kontrolle des eigenen (homologen) Promotors oder nicht in dem Organismus, aus dem er abgeleitet ist, oder weder unter der Kontrolle des eigenen Promotors noch im ursprünglichen Organismus exprimiert wird.

In einer anderen Ausführungsform der Erfindung umfassen die erfindungsgemäßen Nukleinsäure-Moleküle eine unter der Transkriptionskontrolle des hitzeinduzierbaren Promotors stehende Nukleinsäuresequenz, die aus folgenden Sequenzen ausgewählt ist:
(i) einer Nukleinsäuresequenz, die ein Polypeptid mit der Aminosäuresequenz der Trehalose-6-Phosphat-Synthase von *Hansenula polymorpha* kodiert;
(ii) einer Nukleinsäuresequenz wie in SEQ ID NO:6 angegeben;
(iii) einer Nukleinsäuresequenz, die mindestens 80% Identität mit der in SEQ ID NO:6 angegebenen Sequenz aufweist;
(iv) einer Nukleinsäuresequenz, die ein Polypeptid mit der in SEQ ID NO:7 angegebenen Aminosäuresequenz oder mit einer Teilsequenz davon kodiert, wobei das Polypeptid Trehalose-6-Phosphat-Synthase-Aktivität aufweist;
(v) einer Nukleinsäuresequenz, die unter Berücksichtigung der Entartung des genetischen Codes ein Polypeptid mit der in SEQ ID NO:7 angegebenen Aminosäuresequenz oder mit einer Teilsequenz davon kodieren würde, wobei das Polypeptid Trehalose-6-Phosphat-Synthase-Aktivität aufweist;
(vi) einer Nukleinsäuresequenz, die ein Polypeptid kodiert, dessen Aminosäuresequenz mindestens 80% identisch mit der in SEQ ID NO:7 angegebenen Aminosäuresequenz ist.

Vorzugsweise weist die unter (iii) angegebene Nukleinsäuresequenz mindestens 90% Identität mit der in SEQ ID NO:6 angegebenen Sequenz auf. In einer alternativen Form der erfindungsgemäßen Nukleinsäure-Moleküle kodiert die unter (vi) angegebene Nukleinsäuresequenz ein Polypeptid, dessen Aminosäuresequenz mindestens 90% identisch mit der in SEQ ID NO:7 angegebenen Aminosäuresequenz ist.

Das erfindungsgemäße Nukleinsäure-Molekül kann weiterhin eine Signalpeptid-kodierende Nukleinsäuresequenz umfassen, die den Export des exprimierten Proteins gewährleistet, wobei die Signalpeptid-kodierende Nukleinsäuresequenz vorzugsweise direkt 5 Minuten mit dem zu exprimierenden heterologen Gen verbunden ist. Für die Sekretion und Modifikation vieler eukaryontischer Proteine ist es erforderlich, die Proteinsequenz am N-Terminus mit einer Signalsequenz zu fusionieren, um die Polypeptide in den Sekretionsapparat zu lenken. Hier kommen beispielsweise Komponenten aus dem *S. occidentalis-*Gen *GAM1* und aus einem hormonellen Gen der Krabbe *Carcinus maenas* in Betracht, die für die Sekretion von Hirudin erfolgreich verwendet wurden (Weydemann et al., 1995). Ferner kann das erfindungsgemäße Nukleinsäure-Molekül ein Terminatorelement umfassen, das Signalstrukturen für die RNA-Polymerase enthält, die zur Termination der Transkription führen. Beispiele für verwendbare Terminatorelemente sind der *MOX-* oder der PHO1-Terminator von *H. polymorpha.*

Gegenstand der Erfindung ist ferner eine Wirtszelle, enthaltend einen Expressions vektor, der mindestens ein erfindungsgemäßes Nukleinsäure-Molekül umfasst, wobei die Wirtszelle eine prokaryontische oder eine eukaryontische Zelle ist. Beispielsweise kann die eukaryontische Zelle eine Pflanzenzelle sein. Vorzugsweise ist die eukaryontische Zelle eine Pilzzelle, besonders bevorzugt eine Hefezelle. Als Wirtszelle zur Ausführung der vorliegenden Erfindung kommen insbesondere Pilze in Betracht, beispielsweise filamentöse Pilze wie etwa *Aspergillus, Neurospora, Mucor, Trichoderma, Acremonium, Sordaria* und *Penicillium* oder Hefen wie *Saccharomyces, Hansenula, Pichia, Kluyveromyces, Schwanniomyces, Yarrowia, Arxula, Trichosporon* und *Candida.*

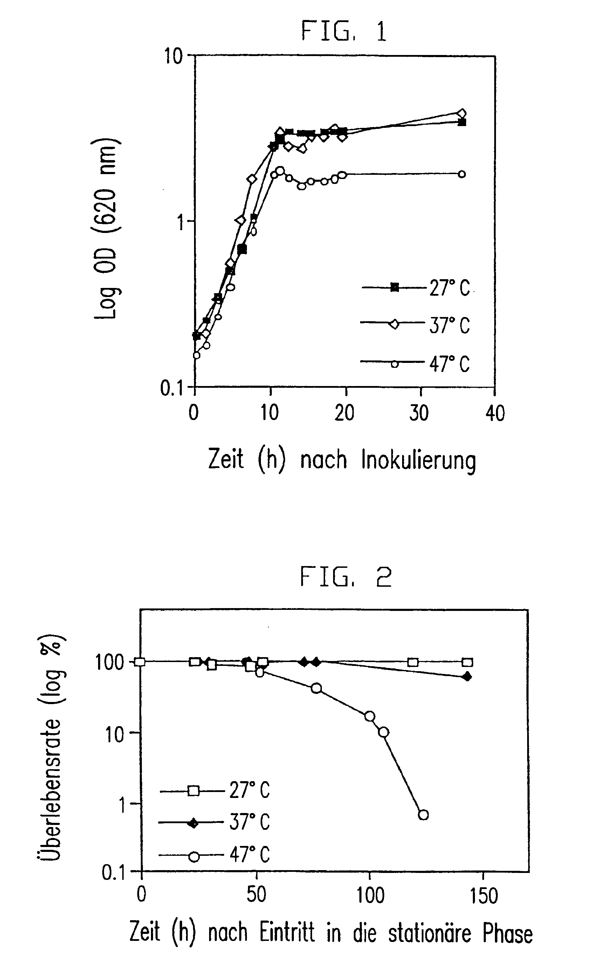

Bei der am stärksten bevorzugten Ausführungsform der Erfindung ist die Hefezelle eine fakultativ methylotrophe *Hansenula-*Hefe, bevorzugt *Hansenula polymorpha. H. polymorpha* ist eine hitzetolerante Hefezelle und gehört zu einer kleinen Gruppe von sogenannten methylotrophen Hefen, die Methanol als Kohlenstoff- und Energiequelle nutzen können. *H. polymorpha* wurde durch Inkubation bei 37°C aus Bodenproben isoliert (Levine und Cooney, 1973). Die hohe Temperatur, bei der *H. polymorpha* noch wächst und Protein produziert, ermöglicht die Ausmerzung anderer, nicht erwünschter Organismen. Es hat sich nämlich gezeigt, daß *H. polymorpha* nicht nur eine sehr hohe optimale Wachstumstemperatur im Bereich um 37°C hat, sondern auch Hitze von ca. 50°C unbeschadet übersteht (siehe Figur 1). Die Vitalität von *H. polymorpha* nach Eintritt in die stationäre Phase nimmt selbst bei 47°C erst nach ca. 50 Stunden ab (Figur 2).

Ein weiterer Gegenstand der vorliegenden Erfindung ist ein Expressionsvektor, der mindestens ein erfindungsgemäßes Nukleinsäure-Molekül umfaßt. Ein solcher Expressionsvektor kann neben einem hitzeinduzierbaren Promotor auch andere Nukleinsäuresequenzen enthalten, beispielsweise eine Sequenz, die ein Polypeptid kodiert, ein Selektionsmarkergen, einen Replikationsursprung für *E. coli,* etc.

Die vorliegende Erfindung stellt ebenfalls einen Kit bereit, der:
(a) einen erfindungsgemäßen Expressionsvektor, der zum Hineinklonieren einer Nukleinsäure geeignet ist, welche ein rekombinantes Protein kodiert, und
(b) eine zur Induktion des hitzeinduzierbaren Promotors und zum Herstellen des rekombinanten Proteins geeignete Wirtszelle
   umfaßt. "Klonieren" soll alle im Stand der Technik bekannten Klonierungsmethoden umfassen, die hier zum Einsatz kommen könnten, die jedoch nicht alle im einzelnen beschrieben werden, weil sie zum selbstverständlichen Handwerkszeug des Fachmanns gehören.

Ferner wird von der Erfindung ein Kit bereitgestellt, der
(a) einen Expressionsvektor und
(b) eine Wirtszelle, die zur Induktion des hitzeinduzierbaren Promotors und zur Herstellung eines von einer kodierenden Sequenz unter der Transkriptionskontrolle des hitzeinduzierbaren Promotors kodierten Proteins geeignet ist,
   umfaßt.

Die erfindungsgemäßen Nukleinsäure-Moleküle, Wirtszellen, Expressionsvektoren und Kits können zur rekombinanten Expression eines Gens unter der Kontrolle des hitzeinduzierbaren Promotors oder zum Herstellen von einem oder mehreren Proteinen verwendet werden.

Unter "rekombinanter Expression in einer geeigneten Wirtszelle" sollen alle im Stand der Technik bekannten Expressionsmethoden in bekannten Expressionssystemen verstanden werden, die hier zum Einsatz kommen könnten, jedoch nicht alle im einzelnen beschrieben werden, weil sie zum selbstverständlichen Handwerkszeug des Fachmanns gehören.

Gegenstand der Erfindung ist ferner ein Verfahren zum Herstellen von einem oder mehreren Proteinen, umfassend:
(i) Hineinklonieren von mindestens einer Nukleinsäure, die ein rekombinantes Protein kodiert, in einen erfindungsgemäßen Expressionsvektor, so daß die hineinklonierte Nukleinsäure unter der Transkriptionskontrolle des hitzeinduzierbaren Promotor steht;
(ii) Einführen des in (i) erhaltenen Expressionsvektors in eine zur Induktion des hitzeinduzierbaren Promotors und zum Herstellen des rekombinanten Proteins geeignete Wirtszelle;
(iii) Kultivieren der in (ii) erhaltenen Wirtszelle;
(iv) Induzieren des hitzeinduzierbaren Promotors in an sich bekannter Weise.

Enthält der erfindungsgemäße Expressionsvektor eine unter der Transkriptionskontrolle des hitzeinduzierbaren Promotors stehende Sequenz, die ein Polypeptid kodiert, so umfaßt das erfindungsgemäße Verfahren zum Herstellen von einem oder mehreren Proteinen die folgenden Schritte:
(i) Einführen eines Expressionsvektors in eine zur Induktion des hitzeinduzierbaren Promotors und zum Herstellen des rekombinanten Proteins geeignete Wirtszelle;
(ii) Kultivieren der in (i) erhaltenen Wirtszelle;
(iii) Induzieren des hitzeinduzierbaren Promotors in an sich bekannter Weise.

Die Erfindung wird nun in der Folge unter Bezugnahme auf die Figuren näher erläutert, wobei die Figuren das folgende zeigen:
Figur 1 zeigt Wachstumskurven *von H. polymorpha* bei 27°C, 37°C und 47°C.
Figur 2 zeigt die Vitalität nach Eintritt in die stationäre Phase bei 27°C, 37°C und 47°C.
Figur 3A zeigt einen Northern Blot von RNA aus *H. polymorpha* Wildtyp nach Hitzeschock von 27°C auf 47°C und anschließender Abkühlung auf 27°C. Die Zellen wurden in YDP-Medium bei 27°C bis zur frühen exponentiellen Phase kultiviert; sodann wurde die Temperatur auf 47°C erhöht (Zeit 0) und nach 120 Minuten wieder auf 27°C gesenkt.
Figur 3B zeigt einen Western Blot für das Tps1-Protein (Tps1 p) aus *H. polymorpha* nach Hitzeschock von 27°C auf 47°C und anschließender Abkühlung auf 27°C (siehe Figur 3A), aus dem eine Korrelation der Zunahme der *TPS1-*mRNA mit der Zunahme des Tps1-Proteins (Tps1p) ersichtlich ist.
Figur 3C stellt die intrazelluläre Trehalosekonzentration und die Trehalose-6-Phosphat-Synthaseaktivität gegen Zeit bei *H. polymorpha* nach Hitzeschock von 27°C auf 47°C und anschließender Abkühlung auf 27°C dar (siehe Figur 3A). Die offenen Kreise symbolisieren die intrazelluläre Trehalosekonzentration, die ausgefüllten Quadrate die Trehalose-6-Phosphat-Synthaseaktivität. Daraus ist eine Korrelation der Zunahme der *TPS1-*mRNA mit der Zunahme der Trehalose-6-Phosphat-Synthaseaktivität und der intrazellulären Trehalosekonzentration ersichtlich.
Figur 4 zeigt drei Balkendiagramme, in denen die Trehalose-6-Phosphat-Synthaseaktivität (weiße Balken) und die intrazelluläre Trehalosekonzentration (schwarze Balken) in bei 27°C (A), 37°C (B) und 47°C (C) und Glukoseentzug kultivierten Zellen von *Hansenula polymorpha* nach 7, 10, 17 und 36 Stunden dargestellt werden. Die Trehaloseakkumulation korreliert mit der Zunahme der Trehalose-6-Phoshat-Synthaseaktivität (Figur 4A), der *TPS1-*mRNA (Figur 4B) und des Tps1-Proteins (Tps1p) (Figur 4C).

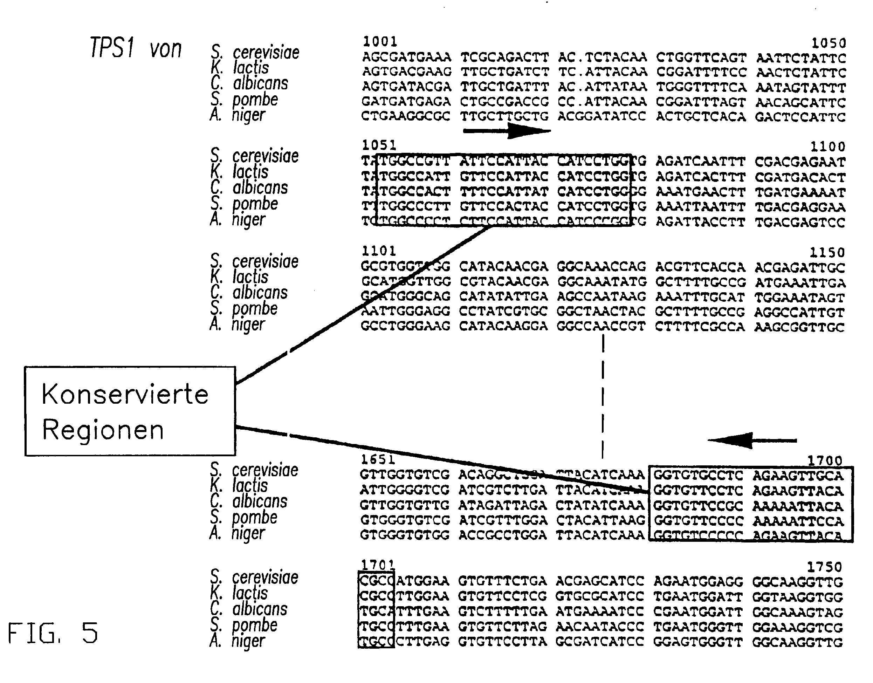

Figur 5 zeigt die Homologie bestimmter DNA-Sequenzbereiche von Trehalose-6-Phosphat-Synthase verschiedener Organismen.
Figur 6 zeigt die DNA-Sequenz des TPS1-Gens von *H. polymorpha* (SEQ ID NO:8) sowie die davon abgeleitete Aminosäuresequenz (SEQ ID NO:6). Die Hitzeschock-Elemente in der Promotor-Sequenz sind durch Unterstreichung markiert.
Figur 7 zeigt das Plasmid pC11, ein Derivat von pM1 (M. Suckow, persönliche Mitteilung), das durch Insertion des lacZ-Gens in den Polylinker von pM1 erhalten wurde. Das Plasmid enthält die HARS1-Sequenz (*H*. *polymorpha* Autonomously Replicating Sequences), den ori (Replikationsursprung) aus pBR322, ein Ampicillin-Resistenzgen, das URA3-Gen für die Propagation und Selektion in *H. polymorpha* und in *E*. *coli* sowie ein MOX-Terminator hinter dem lacZ-Gen zur Beendigung des Transkriptionsvorgangs.
Figur 8 zeigt das Plasmid pC11-FMD, welches durch Insertion des FMD-Promotors vor dem lacZ-Reportergen von pC11 erhalten wurde.
Figur 9 zeigt das Plasmid pC11-TPS1, welches durch Insertion des TPS1-Promotors vor dem lacZ-Reportergen von pC11 erhalten wurde.
Figur 10 zeigt einen Vergleich der Aktivitäten von FMD- (A) und TPS1-Promotor (B) bei 30, 37 und 44°C in drei verschiedenen Kohlenstoffquellen (2% Glukose, 2% Glycerol oder 2% Methanol).
Figur 11 zeigt das in Beispiel 4 eingesetzte Plasmid pTPS1ConphysMT. MOX-T = MOX-Terminator, Conphys = Conphys3-Gen, TPS1 = TPS1-Promotor von *Hansenula polymorpha,* HARS = *H. polymorpha* Autonomously Replicating Sequences, tet = Tetracyklin-Resistenzgen, URA3 = URA3 von *S. cerevisiae,* amp = Ampicillin-Resistenzgen

### Beispiele

### Materialien und Methoden:

| **Spezielle Reagenzien und Materialien** | |
|---|---|
| Bio 101, Vista, USA | Geneclean II Kit |
| BioRad Lab., München, D | BioRad Protein Assay (Bradford) |
| Boehringer, Mannheim, D | GOD/POD Kit für Glukosebestimmung, Ethanol Kit, "COMPLETE" Proteinase Inhibitor Cocktail Tabletten |
| Fluka Chemie AG,Buchs,CH | Cycloheximid(Actidion),SDS, D+Trehalose, PEP, TRICIN, NADH, Folin-Ciocalteu's phenol reagent |
| ICN Biochemicals, Ohio, USA | "Liquigel" 40% Acrylamid/N'N'-methylen-bisacrylamid (37,5:1) |
| Kodak, New York, USA | BIOMAX MR scientific imaging film |
| Mediatech, Herndon, USA | Geneticin G418-Sulphat (Antibiotikum) |
| Perkin Elmer Applied Biosystems, | DNA Sequencing Kit Forest City, USA |
| Pharmacia Biotech, Schweden | Nap-10 Säulen (mit Sephadex G-25), alle ver-wendeten Restriktionsenzyme, Taq-Polyme-rase |
| Qiagen GmbH, D | Plasmid Midi Kit (50) |
| Schleicher + Schuell, Dassel, D | Protran BA 83 0,2 µm/ Ø 82 mm (Nitrozellu-lose-Rundfilter), Protran BA 83 0,2 µm (Transfermembran für Blots) |
| Sigma, St.Louis, USA | Monoclonal goat anti-rabbit Immunoglobu-lins(alkaline phosphatase conjugate), Treha-lase aus Schweinenieren (Cat. No. T-8778), UDPG, Glukose-6-P, LDH, Pyruvatkinase |
| Stratagene, La Jolla, USA | Prime-It II Kit (random primer labelling kit), |
| | NucTrap-Säulen (probe purification columns inkl push column beta shield device) |
| US Biological, Swampscott, USA | Bacteriological Agar, YPD broth enhanced formulation W/Peptone X, LB broth Miller |
| **Verwendete Apparaturen** | |
| Elektroporationsgerät | *E. coli* Pulser, BioRad Laboratories, Hercules USA |
| HPLC | DIONEX DX-300, DIONEX, Sunnyvale, USA |
| Kühlzentrifugen | Centrikon H-401, Kontron Instr. AG, Zürich, CH IEC Centra GP8R, Brouwer, Luzem, CH Biofuge 17RS, Heraeus Sepatech, D |
| PCR-Gerät | Progene, Techne, Cambridge, GB |
| Phosphoimager | GS 250 Molecular Imager (inkl. restliche Aus-rüstung), BioRad Laboratories, Hercules, USA |
| Photometer | Anthos 2001 (für Mikrotiterplatten), Anthos Labtec Instruments, Salzburg, A Shimadzu UV-160A, J |
| Sequenziergerät | ABI PRISM 301 Genetic Analyzer, Perkin Elmer Applied Biosystems, Foster City, USA |

### Bakterienstamm und Kulturbedingungen

Zur Klonierung des *TPS1-*Gens von *H. polymorpha* wurde der *E. coli*-Stamm DH5α (F'endA1*hsd*R17rₖmₖ+*supE44thi-1recA1gyra relA(lacZYA-argF)* U169(φ80Δ(lacZ)M15) (Gibco BRL, Gaithersburg MD, USA) verwendet, wobei nach Standardprotokollen (Sambrook *et. al.,* 1989) gearbeitet wurde. Medium für *E. coli wurde* ebenfalls nach Standardrezept (Sambrook *et al.,* 1989) hergestellt.

### Isolierung von Plasmid-DNA aus E. coli (STET-Prep.)

Plasmid-DNA wurde nach einem modifizierten Protokoll von Sambrook *et. al.* (1989) isoliert. Mit einem Spatel wurde Zellmaterial von einer Platte abgekratzt, in 500 µl STET (8% [w/v] Saccharose, 5% [v/v] Triton X-100,50 mM EDTA, 50 mM Tris-HCl, pH 8,0) mit 35 µl Lysozym (10 mg/ml) gegeben und gemischt. Anschließend wurden die Proben 1 min 40 s bei 100°C gekocht und 10 min lang bei 20.000 g und 4°C zentrifugiert.
Ca. 400 µl Überstand wurden abpipettiert und die DNA mit 400 µl Isopropanol gefällt. Nach einer 10minütiger Zentrifugation bei 20.000 g und 4°C wurde der gesamte Überstand verworfen und das DNA-Pellet einmal mit eiskaltem 70% [v/v] Ethanol gewaschen. Schließlich wurde die DNA bei Raumtemperatur getrocknet und in 50-70 µl TE (10 mM Tris-HCl, pH 8,0, 1 mM EDTA, pH 8,0) aufgenommen.

### Hefestamm und Kulturbedingungen

Bei dem verwendeten Hefestamm handelt es sich um einen *Hansenula polymorpha* Wildtyp (erhalten von P. Piper, London (1994)). Stammkulturen wurden auf YPD-Agar (2% [w/v] Glukose, 2% [w/v] Bactopepton, 1 % [w/v] Hefeextrakt, 2% [w/v] Agar) herangezogen und alle 6 Wochen neu angelegt. Sie dienten als Inokulum für YPD-Flüssigkulturen (gleiche Zusammensetzung wie YPD-Agar, aber ohne 2% [w/v] Agar).

Für die Versuche in den Beispielen 3 und 4 wurde der Stamm *H. polymorpha* RB11 (odc1 Orotidin-5-phosphat-Decarboxylase-defizienter (Uracil-auxotropher) *H. polymorpha-Stamm* (Weydemann *et al.,* 1995)) verwendet. Das verwendete Vollmedium enthielt 2% Glukose oder Glycerin, 1% Hefeextrakt und 2% Bacto-Peptone, das Selektionsmedium 0,17% Yeast Nitrogen Base, 0,5% Ammoniumsulfat, 2% Glukose oder Glycerol, 38,4 mg/l Arginin, 57,6 mg/l Isoleucin, 48 mg/l Phenylalanin, 57,6 mg/l Valin, 6 mg/ml Threonin, 50 mg/l Inositol, 40 mg/l Tryptophan, 15 mg/l Tyrosin, 60 mg/l Leucin, 4 mg/l Histidin. Uracil fehlt im Selektionsmedium.

Zur Anzucht von Zellkulturen wurden autoklavierte Flüssigmedien mit Stammkultur beimpft und über Nacht in Schüttelinkubatoren bei - je nach Experiment - 27°C, 37°C oder 47°C inkubiert.

### OD-Bestimmung der H. polymorpha-Zellkulturen

Um die OD (= optische Dichte) zu bestimmen, wurden 200 µl (evtl. mit YPD geeignet verdünnter) Zellkultur in ein Vial einer Mikrotiterplatte gegeben und mit einem Anthos 2001 Photospektrometer bei 620nm gemessen. Als Blank wurden 200 µl YPD eingesetzt.

### Wachstums- und Hitzeschockexperimente mit H. polymorpha

Übemach-Kulturen wurden jeweils verwendet, um YPD-Medium in Erlenmeyern zu inokulieren. Dabei wurde stets darauf geachtet, daß diese Vorkultur bei jener Temperatur angezogen wurde, bei der später auch das Experiment begonnen wurde (27°C für Hitzeschocks, 27°C, 37°C oder 47°C für Wachstumsexperimente).

Für Wachstumsexperimente wurde jeweils auf eine Anfangs-OD₆₂₀ von 0,2 inokuliert, wobei die Zellkulturen dauernd in Schüttelinkubatoren (Multitron) gehalten wurden. Bei Hitzeschock-Experimenten wurde hingegen auf einen Anfangs-OD von 0,05 inokuliert. Man liess die Kultur bei 27°C bis zur OD₆₂₀ 0,4 (ca. 1-1,5 x 10⁸ Zellen pro ml Kultur) weiter wachsen, bevor der Hitzeschock auf 47°C in einem Wasserbad mit Schüttelfunktion (Aquatron) durchgeführt wurde. Danach wurden während weiterer 2 h Proben entnommen. Anschließend wurde die Zellkultur in einem zweiten Wasserbad 1 h lang wieder auf 27°C abgekühlt.

### Transformation von H. polymorpha durch Elektroporation

100 ml YPD wurden mit 5 ml einer dichtgewachsenen Übemacht-Kultur beimpft. Die Kultur wurde bei 37°C bis zu einer OD₆₀₀ von 0,8-1,2 geschüttelt (ca. 3 Std.). Die Zellen wurden durch Zentrifugation bei 3000 rpm geerntet und in 20 ml KPᵢ-Puffer (50 mM/pH 7,5) resuspendiert. Nach Hinzufügen von 0,5 ml DTT und 15minütigem Schütteln bei 37°C wurden die Zellen bei 2500 rpm abzentrifugiert und 2x mit STM-Puffer (270 mM Saccharose, 10 mM TrisCl, 1 mM MgCl₂, pH 7,5) gewaschen. Danach wurden die Zellen in 0,25 ml STM-Puffer aufgenommen und 60 µl-Aliquots bei -70°C gelagert. Zur Transformation mit rDNA-Integrationsvektoren wurde die Plasmid-DNA zunächst mit *X*hol oder Sacl linearisiert. 0,1-1 µg der linearisierten Plasmid-DNA wurden mit frische, auf Eis aufgetauten kompetenten Zellen gemischt, diese Ansätze dann in 2mm eine Küvette gegeben. Die Transformation erfolgte in einem Gene-Pulser (Bio-Rad, München) bei 2,0 kV, 25 µF und 200 Ohm. Anschließend wurden die Zellen zur Erholung in 1 ml YPD für 1 Std. bei 37°C inkubiert und dann auf Selektions-Medium ausplattiert. Nach 2-4 Tagen Inkubation bei 37°C wurden makroskopische Kolonien sichtbar.

### Bestimmung der Glukosekonzentration im Medium

Die Glukosekonzentration im Medium wurde mittels die GOD-Methode (GOD/POD Kit, Böhringer) bestimmt. Proben wurden 1:200 mit Wasser verdünnt. Je 10 µl davon wurden mit 190 µl 1% (w/v) GOD-Enzymlösung (in Pulverform im Kit mitgeliefert) versetzt und bei 27°C ca. 25 min inkubiert. Als Standard diente die im Kit mitgelieferte Glukoselösung, wovon ebenfalls 10 µl (0,91 µg Glukose) eingesetzt wurden. Die Absorption wurde im Anthos 2001 Spektrophotometer bei 405 nm gemessen.

### Extraktion und quantitativer Nachweis von Trehalose

### Extraktion von Trehalose

1-10 ml Zellkultur wurden über einen Glasfaserfilter (Whatman GF/C) abgenutscht und dreimal mit Wasser gewaschen. Der Filter wurde in ein Eppendorf mit 1 ml Wasser gegeben und 30 s gevortext, bevor er dann sorgfältig ausgedrückt und entfernt wurde. Anschließend wurde die Zellsuspension 10 min lang im Wasserbad gekocht. Um den Überstand vollständig von Zellmaterial zu befreien, wurde er dreimal bei 20.000 g zentrifugiert.

### Trehalosebestimmung mittels HPLC

Die extrahierten Zucker wurden durch eine Anionenaustauschersäule (DIONEX Carbo-Pac PA1-Säule, 4 x 250 mm) getrennnt und amperometrisch an einer Goldelektrode detektiert (PED = Pulsed electrochemical detector). Der eluierende Gradient setzte sich folgendermassen zusammen:

| Zeit (min) | H₂O | H₂O | 1 M Na-Acetat | 1 M NaOH |
|---|---|---|---|---|
| 0,0 | 45% | 45% | 0% | 10% |
| 3,5 | 40 % | 39% | 0 % | 21 % |
| 4,5 | 35% | 35% | 20% | 10% |
| 5,0 | 45% | 45% | 0% | 10% |
| 14,0 | 45% | 45% | 0 % | 10 % |

Unter diesen Bedingungen ergab sich für Trehalose eine Retentionszeit von ca. 3,7 min. Es wurden jeweils 20 µl Probe eingespritzt. Als Standard diente eine Lösung aus 0,1 mg/ml Trehalose.

### Trehalosebestimmung mittels Enzymassay

Als Alternative zur teureren HPLC-Methode wurde zum Teil ein genauso verlässlicher Enzymassay angewendet (Parrou und François, 1997, modifiziert): 25 µl Trehaloseextrakt wurden mit 12,5 µl Trehalase (Sigma) und 37,5 µl Pufferlösung (0,2 M Natriumacetat, 0,03 M CaCl₂, pH 5,7) gemischt und im Wasserbad 5 h lang bei 37°C inkubiert.
Dies führte zum vollständigen Abbau von Trehalose zu zwei Einheiten Glukose. Nach kurzer Zentrifugation wurden die Proben 3 min lang bei 95°C inkubiert und schließlich nochmals 5 min bei 20.000 g zentrifugiert. Die Trehalosekonzentration wurde indirekt via Glukosekonzentrationsbestimmung (GOD/POD-Kit, siehe oben) bestimmt. Dazu wurden 10 µl dieses Überstandes eingesetzt.

### Proteinbestimmung

### Proteinbestimmung nach Peterson (leicht modifiziert) (Peterson (1977))

Für die Bestimmung der Gesamtproteinkonzentration einer Zellkultur wurde 1 ml Zellsuspension in 1 ml 10% (w/v) TCA gefällt und 10 min lang bei 3000 g zentifugiert. Der Überstand wurde mit einer an einer Wasserstrahlpumpe angeschlossener Pasteur-Pipette abgesogen und das Sediment in 1 ml 1N PCA gewaschen. Das Pellet wurde anschließend in 5-12 ml (je nach OD der zu untersuchenden Zellkultur) einer Lösung aus 0,8 N NaOH : 10% (w/v) SDS (1:1) aufgenommen und mindestens 1 h bei 60°C inkubiert. 200 µl dieser Suspension wurden mit 600 µl sechsfach verdünntem CTC-Reagens (10% Na₂CO₃, 0,1% CuSO₄ 5H₂O, 0,2% KNa-Tartrat) versetzt. Nach genau 10 min wurden 200 µl sechsfach verdünntes Folin-Ciocalteu's Reagens dazugegeben und kurz gemischt. Nachdem die Proben 30 min lang im Dunkeln stehengelassen worden waren, wurde die Absorption bei 750 nm gemessen, wobei BSA als Standard diente.

### Proteinbestimmung nach Bradford (1976)

Für die Bestimmung der Proteinkonzentration in zellfreiem Extrakt wurden 100 µl eines geeignet verdünnten Extraktes mit 700 µl Wasser vermischt. Danach wurden 200 µl BioRad Protein Assay Reagens (Bradford) gegeben und kurz geschüttelt (Vortex). Die Absorption wurde bei 595 nm gemessen, wobei BSA als Standard diente.

### Enzymaktivitätsmessungen

### Herstellung permeabilisierter Zellen

Die Enzymaktivität der Trehalose-6-Phosphat-Synthase (Tre6P-Synthase) wurde in permeabilisierten Zellen (De Virgilio *et al.,* 1991) gemessen. Dazu wurden 1-6 ml Zellen abgenutscht (auf GF/C Glasfaserfilter, Whatman), zweimal mit eiskaltem Wasser gewaschen und in 1 ml Lysierungspuffer (0,2 M TRICIN, pH 7,0,0,05% [v/v] Triton X-100) durch Vortexen resuspendiert. Nach Entnahme der Filter wurden die Eppendorf-Röhrchen in flüssigem Stickstoff gefroren und bei -20°C aufbewahrt. Vor der Durchführung der Messung wurden die Zellen 3 min lang im Wasserbad bei 30°C aufgetaut. Danach wurden sie zweimal in 0,2 M TRICIN (pH 7,0) gewaschen, wobei dazwischen je 20 s bei 4°C und 8000 rpm (Biofuge 17RS) zentrifugiert wurde. Die Zellen wurden schließlich in 600 µl 0,2 M TRICIN (pH 7,0) resuspendiert.

### Trehalose-6-Phosphat-Synthaseaktivität

Die Tre6P-Synthaseaktivität wurde gemäß dem gekoppelten Enzymassay nach Hottiger *et al.* (1987) bei 50°C bestimmt, wobei stets 60 µl permeabilisierte Zellen eingesetzt wurden. Sowohl Substrat- (ohne Glukose-6-P) als auch Enzymblanks (ohne permeabilisierte Zellen) wurden als Kontrollen mitgenommen.

### Western Blot-Analysen

### Proteinextraktion durch Zellaufschluss

5-15 ml Zellkultur wurden 5 min lang bei 4°C und 3000 rpm (IEC Centra GP8R) zentrifugiert und der Überstand wurde abdekantiert. Das Pellet wurde in 1 ml Wasser aufgenommen und in ein Sarstedt-Röhrchen (mit Schraubverschluss) überführt. Nach einer 10 s langen Zentrifugation wurde der Überstand abpipettiert und das Pellet abgewogen, wobei als Tara ein leeres Röhrchen diente. Pro mg Pellet wurde 1 µl 0,2 M TRICIN-Puffer (pH 7,0; mit Proteinase Inhibitoren [2 Tabs/ 25 ml]) dazugegeben und resuspendiert. Glaskügelchen wurden bis knapp unter den Flüssigkeitsmeniskus eingefüllt, woraufhin die Sarstedt-Röhrchen im Kühlraum in ein Zellaufschlussgerät (Fastprep FP120) fest eingespannt wurden. Dieses wurde bei Stärke 6.0 zweimal 30 Sekunden lang laufengelassen, was einen >90%igen Zellaufschluß ergab. Von diesem Zeitpunkt an wurde streng darauf geachtet, daß die Proben stets gut gekühlt blieben. Mit einer Nadel wurde ein kleines Loch in das Sarstedt-Röhrchen gebohrt. Dieses wurde auf ein Glasröhrchen aufgesetzt und bei 4°C und 100 g zentrifugiert, wodurch der Extrakt von den Glaskügelchen getrennt wurde. Die Sarstedt-Röhrchen wurden anschließend einmal mit derselben für den Zellaufschluss gebrauchten Menge TRICIN-Puffer versetzt und wiederum zentrifugiert. Der trübe Extrakt wurde dann in Eppendorfs transferiert und dreimal 10 min lang bei 25.000 g und 4°C (Biofuge 17RS) zentrifugiert, wobei jeweils der Überstand mit den löslichen Proteinen (darunter auch Tre6P-Synthase) weiterverwendet wurde.

### Probenvorbereitung

Die Proteinkonzentration dieser Extrakte wurde nun mit der Bradford-Methode (siehe oben) bestimmt. Gemäß den erhaltenen Werten wurde mit Wasser auf 2,5 µg Proteine/µl verdünnt und zu 4 Volumen dieser Proteinlösung 1 Volumen 5x Probepuffer dazugegeben. Anschließend wurden die Proben 5 min lang bei 95°C denaturiert und entweder direkt für die SDS-Gelelektrophorese verwendet oder eingefroren. Es wurden jeweils 10 µl, also 20 µg Protein, für die Analyse eingesetzt.

Probenpuffer: 1 ml 0,5 M Tris-HCl, pH 6,8, 0,8 ml Glycerol, 1,6 ml 10%
[w/v] SDS, 0,2 ml 0,05% [w/v] Bromophenolblau, 4 ml
Wasser. Unmittelbar vor Gebrauch wurden 19 Volumen Probenpuffer mit 1 Volumen 2-β-Mercaptomethanol versetzt.

### SDS-Polyacrlamid-Gelelektrophorese (SDS-PAGE)

Für die Trennung der Proteine nach ihrem Molekulargewicht wurde das System nach Laemmli *et al.* (1970) verwendet. Als Trenngel wurde ein 10%iges, als Sammelgel ein 4%iges Acrylamidgel (Totalgrösse 10X10 cm) mit folgender Zusammensetzung hergestellt:

| | |
|---|---|
| Trenngel: | 2,5 ml 40% (w/v) Acrylamid/Bisacrylamid, 2,5 ml 1,5M Tris-HCl, pH 8,8, 100 µl 10% (w/v)SDS, 4,95 ml Wasser 50 µl 10% (w/v) Ammoniumpersulfat, 5 µl TEMED |
| Sammelgel: | 1 ml 40% (w/v) Acrylamid/Bisacrylamid, 2,5 ml 0,5M Tris-HCl, pH 6,8, 100 µl 10% (w/v) SDS, 6,4 ml Wasser 50 µl 10% (w/v) Ammoniumpersulfat, 10µl TEMED |
| 5x Laufpuffer | 15 g Tris, 72 g Glycin, 5 g SDS, H₂O ad 1 l. Der pH-Wert sollte (ohne weiteres Einstellen) bei ca. 8,3 liegen. |

Es wurden jeweils 20 µg Protein auf das Gel geladen. Als Standard diente der "Kaleidoscope prestained standard" von BioRad, der sich wie folgt zusammensetzte: Myosin (204 kDa), β-Galactosidase (121 kDa), BSA (78 kDa), Carboanhydrase (39 kDa), Soja-Trypsinininhibitor (30 kDa). Die Gelelektrophorese wurde ca. 1 h (aber höchstens solange, bis die Probenfront den unteren Rand des Gels erreicht hatte) bei einer konstanten Spannung von 200 V durchgeführt. Diese Gele wurden dann entweder mit 0,1% (w/v) Coomassie Blue R250 in 10% (v/v) Essigsäure / 50% (v/v) Ethanol angefärbt (und nach ca. 1 h mit 10% (v/v) Essigsäure, 20% (v/v) Ethanol entfärbt) oder auf Nitrozellulose geblottet (siehe nächsten Abschnitt).

### Immunoblotting

Die SDS-PAGE-Gele wurden anschließend in einem Blottinggerät (Scieplas) mit Transblot-Puffer (250 mM Tris, 1250 mM Glycine, 15% (v/v) Methanol) 1 h 15 min lang bei 40 V und 4°C auf Nitrozellulose geblottet.

### Immunfärbung

Die Nitrozellulose-Membran wurde zuerst mindestens 1 h in einer Sättigungslösung aus 3% (w/v) BSA in TBS (TBS: 20 mM Tris, 500 mM NaCl, pH mit HCl auf 7,5 eingestellt) belassen, woraufhin 5 min mit TTBS (TTBS: Gleich wie TBS, aber mit 0,05% Tween-20) gewaschen wurde. Danach wurde über Nacht bei 4°C ein polyklonaler Anti-Tps1 p-Antikörper aus Kaninchen (1:50 verdünnt mit 1 % [w/v] BSA in TTBS) (Eurogentec, Belgien) appliziert, der an das auf der Nitrozellulose vorhandene Tps1-Protein (Tps1p) aus *H. polymorpha* binden soll.

Der Nitrozellulose-Blot wurde anschließend zweimal 5 min lang mit TTBS gewaschen und 1 h 30 min lang mit einem monoklonalen Anti-Kaninchen Antikörper mit gekoppelter alkalischer Phosphatase (1:10.000 verdünnt mit 1% [w/v] BSA in TTBS) inkubiert. Danach wurde wiederum zweimal 5 min lang mit TTBS und einmal 5 min lang mit TBS gewaschen. Um die Färbung der Banden zu entwickeln, wurde 1 ml 10x Farbentwicklungspuffer (100 mM Tris-HCl, pH 9,5, 1 mM MgCl) 1:10 mit Wasser verdünnt und mit 45 µl NBT (75 mg/ ml 70% [v/v] DMF) und 35 µl X-Phosphat (50 mg 5-Brom-4-chlor-3-indolyl-phosphat-toluidinium-Salz/ml DMF) versetzt. Die Membranen wurden mit dieser Mischung 20 min lang (oder bis die Banden gut sichtbar wurden) im Dunkeln inkubiert, bevor sie - um die Reaktion zu stoppen - mit Wasser gewaschen wurden.

### Kolonie-PCR mit Zellen von H. polymorpha

Kolonie-PCR wurde nach einem Protokoll von Huxley *et al.* (1990, abgeändert) durchgeführt: Einzelne Kolonien wurden mit einer gelben Pipettenspitze aufgenommen und in einem PCR-Röhrchen abgestrichen. Die Röhrchen wurden dann in einem Mikrowellenofen bei voller Leistung 2 min lang erhitzt. Schließlich wurden jeweils 25 µl PCR-Mix (0,2 µl Taq-Polymerase, 2,5 µl 10x PCR-Puffer, 2,5 µl 25 mM MgCl₂, 0,5 µl 10 mM dNTP, je 0,5 µM Endkonzentration an Primer und Wasser ad 25 µl) hinzugegeben und die Zellen darin resuspendiert. Gleich danach wurden die Röhrchen in das auf 92°C vorgewärmte PCR-Gerät gestellt und das Programm gestartet.

### Northern Blot-Analyse

RNA wurde nach einem Protokoll von Piper (1994, angepaßt) aus *H. polymorpha* extrahiert. Dazu wurden 40 ml logarithmische oder 20 ml stationäre Zellkultur gesammelt und (bei Hitzeschockexperimenten) sofort durch Zugabe von eiskaltem, sterilem DEPC-Wasser abgekühlt. Die Zellen wurden dann abzentrifugiert und mit sterilem DEPC-Wasser nochmals gewaschen. Das nach der Zentrifugation und Verwerfung des Überstandes erhaltene Pellet wurde bei -20°C aufbewahrt. Dem Pellet wurden dann nach dem Auftauen 1-2 g Glaskügelchen, 2 ml RNA-Extraktionspuffer (20 mM Tris-HCl, pH 8,5,10 mM Na2-EDTA, 1 % [w/v] SDS) und 2 ml Phenol zugegeben. Diese Mischung wurde dann ununterbrochen 5 min lang bei Raumtemperatur gevortext und anschließend 5 min bei 3500 rprn (IEC Centra GP8R) zentrifugiert. Die obere, wässrige Phase wurde in ein neues Röhrchen, das ein gleiches Volumen an Phenol/Chloroform (1:1) enthielt, überführt. Die Suspension wurde 1 Minute lang gevortext, 5 min bei 3500 rpm zentrifugiert und der Überstand in ein neues Röhrchen, das ein gleiches Volumen an Chloroform enthielt, gegeben. Wiederum wurde 1 min gevortext, 2 min bei 3500 rpm zentrifugiert und der Überstand in 15 ml Corex-Glasröhrchen transferiert. Es wurde 6 M Ammoniumacetat bis auf eine Endkonzentration von 1 M Ammoniumacetat und anschließend 2 Volumen Ethanol (eisgekühlt) hinzugefügt und die Röhrchen mindestens 20 min lang ins Tiefkühlfach bei -20°C inkubiert. Die RNA wurde dann 15 min lang bei 7500 g und 4°C abzentrifugiert. Der Überstand wurde abdekantiert und die Röhrchen auf einem Saugpapier getrocknet. Die Pellets wurden danach in 1 ml TE aufigenommen und die RNA durch Zugabe von 3 M Natriumacetat (bis auf eine Endkonzentration von 0,2 M) und 2,5 Volumen eiskaltem Ethanol gefällt. Nach einer 15minütigen Zentrifugation bei 7500 g und 4°C wurde das Pellet mit eiskaltem 70% (v/v) Ethanol gewaschen und bei Raumtemperatur getrocknet. Die RNA wurde schließlich in 400 µl TE resuspendiert.

### Probenvorbereitung

Für die Northern Blot-Analyse (nach Sambrook et al., 1989) wurden pro Probe 50 µg RNA im SpeedVac 10-15 min lang eingetrocknet. Anschließend wurde die RNA in 50 µl Probepuffer (Endkonzentrationen: 20 mM MOPS, pH 7,0, 0,5 mM Natriumacetat, 1 mM EDTA, pH 8,0, 2,2 M Formaldehyd, 50% [v/vl Forrnamid) resuspendiert und 10 min lang bei 55°C erhitzt. Zu jeder Probe wurden schließlich 5,5 µl RNA Ladepuffer (10x) und 1 µl Ethidiumbromid-Lösung (1 µl/ml) gegeben.

### Vor- und Hauptgel

Anhand eines Vorgels (1 % [w/v] Agarose und 0,65 M Formaldehyd in MOPS-Puffer aus 40 mM MOPS, pH 7,0, 10 mM Natriumacetat, 2 mM EDTA, pH 8,0) wurde die Integrität der extrahierten RNA getestet und die Ladungsmenge von Auge abgeglichen. Die Hauptgel-Elektrophorese (gleiche Zusammensetzung wie Vorgel) wurde 34 h lang bei 80 V in MOPS-Puffer als Laufpuffer durchgeführt.

### Blotting

Die Gele wurden zuerst zweimal 20 min lang in 10x SSC (1,5 M NaCl, 170 mM Natriumcitrat) gewaschen. Dann wurde die RNA über Nacht durch Kapillartransfer (mit 20 x SSC als Transferpuffer) auf eine Nitrozellulose-Membran (BA 83) geblottet. Anschließend wurde die Membran in 6x SSC gewaschen, zwischen zwei 3MM-Filterpapiere (Whatman) gelegt und in einem Vakuumofen 2 h lang bei 80°C gebacken, wodurch die RNA an die Nitrozellulose fixiert werden konnte.

### Hybridisierung

Die Nitrozellulose-Membran wurde in 10 ml RNA-Hybridisierungslösung (0,5 M NaHPO₄, pH 7,2, 1 mM EDTA, 1% [w/v] BSA, 7% [w/v] SDS) 5 h lang bei 60°C in einem speziellen Ofen (Hybaid) vorhybridisiert. Für die eigentliche Hybridisierung wurden 150 µl der radioaktiv markierten Sonde (insgesamt ca. 1x 10⁷ cpm) in 10 ml RNA-Hybridisierungslösung gegeben und die Membran über Nacht bei 60°C darin inkubiert. Schließlich wurde die überschüssige Radioaktivität zweimal durch 300 ml Waschpuffer (1 mM EDTA, 40 mM Na₂HPO₄, pH 7,2, 1 % [w/v] SDS) 15 min lang bei 60°C weggewaschen. Die Nitrozellulose-Membran wurde auf BIOMAX-Film exponiert.

Phytase-Nachweis

Aus 3 ml Übernacht-Kulturen wurden die *H.* polymorpha-Zellen geerntet und in 200 µl YNB Medium und 1 ml 5%igem Glycerin aufgenommen. Nach 1-2 Tagen Wachstum wurde zuerst die OD₆₀₀ bestimmt. Die Zellen wurden dann abzentrifugiert und 25 µl des Überstandes weiterverwendet. Zu diesem Aliquot wurden 25 µl 5 M NaAc und 50 µl 4-Nitrophenylphosphat hinzugegeben. Der Ansatz wurde 30 min. bei 37°C inkubiert. Die enzymatische Umsetzung des Substrates wurde durch Zugabe von 100 µl 15%iger Trichloressigsäure abgestoppt. Nach Zugabe von 100 µl 1M NaOH erschienen positive Kulturüberstandsproben intensiv gelb gefärbt. Die Gelbfärbung wurde durch OD₄₀₅-Messung im Photometer quantifiziert.

### X-Gal-Overlay-Assay - Nachweis von ß-Galactosidase

Die zu testenden Stämme wurden für 4-6 Stunden in Selektionsmedium bei 37°C kultiviert. Ein 4 µl-Tropfen einer jeden Kultur wurde auf eine Selektionsplatte gegeben und über Nacht bei 37°C inkubiert. Die Platte wurde mit frischen Überschichtungsagar (0,5% Agarose, 0,5 M Na₂HPO₄/NaH₂PO₄ (pH 7); 0,2% SDS; 2% DMF (Dimethylformamid) 2 mg/ml X-gal (o-Nitrophenyl-β-D-Galactopyranoside) bei 70 °C überschichtet. Nach wenigen Minuten zeigten die Klone mit /acZ-Expression eine Blaufärbung.

### Beispiel 1

### Klonierung des TPS1-Gens von H. polymorpha

### Herstellung einer radioaktiven TPS1-Sonde

Aufgrund eines Sequenzenvergleichs der bereits bekannten *TPS1-*Gene aus *S. cerevisiae, S. pombe, K. lactis, Candida albicans* und A. *niger* (siehe Figur 6) konnten aus zwei hochkonsevierten Regionen zwei degenerierte Primer hergestellt werden, die während der PCR (aus 30 Zyklen à 1 min 92°C, 30 s 52°C, 1 min 72°C) mit genomischer DNA aus *H. polymorpha* ein ca. 650 bp Fragment amplifizierten. Die beiden Primer hatten folgende Sequenzen:
F1 (vorwärts): 5' TGGCCVYTNTTCCAYTACCATCCYGG 3' (SEQ ID NO:9)
R1 (rückwärts): 5' GGCRTGBAAYTTYTGHGGHACACC 3' (SEQ ID NO:10)
B = C, G, T H = A, C, T R = A, G
V = A, C, G N = A, C, G, T Y = C, T

Das PCR-Produkt wurde anschließend auf ein präparatives 1 % (w/v) Agarose-Gel geladen und elektrophoretisch aufgetrennt. Die 650 bp Bande wurde ausgeschnitten, mit dem Geneclean II-Kit (Bio 101, Vista, USA) extrahiert und mit radioaktivem [α-³²P]-dCTP markiert. Dazu wurden der Prime-It II-Kit und zur Reinigung die NucTrap-Säulen verwendet. Diese radioaktive Sonde wurde für den *TPS1-*Screen von *H. polymorpha* und für die Northern Blot Analyse eingesetzt.

### Genomische DNA-Bibliothek von H. polymorpha:

Die verwendete genomische DNA-Bibliothek wurde von R. Hilbrands (University of Groningen, NL) zur Verfügung gestellt. Die Herstellung der genomischen DNA Bibliothek ist unkritisch, solange die Fragmente ≥ ca. 2 kb sind. 2-5 kb lange genomische DNA-Fragmente von *H. polymorpha* (möglicherweise ein Vielfaches davon) wurden an der Restriktionsstelle *BamH*l in pHRP2 (7813 bp) kloniert. Dieses Plasmid (Faber *et al.,* 1992) enthält einen *ori* (replication origin) und ein Ampicillin-Resistenzgen zur Vermehrung und Selektion in *E. coli.* Für Transformationen in *H. polymorpha* sind die HARS1-Sequenz *(H. polymorpha* autonomously replicating sequence) und das *S. cerevisiae* LEU2-Gen als Marker, der auch in *H. polymorpha* funktioniert, verantwortlich. Diese Bibliothek enthält ca. 20.000 verschiedene Klone.

### Transformation von E. coli

Die genomische DNA-Bibliothek wurde durch Elektroporation in *E. coli* transformiert (Sambrook *et al.,* 1989) und auf 50 LB+Amp (75 mg/l) Platten (je 2000-4000 Kolonien) ausplattiert. Sie wurden über Nacht bei 37°C inkubiert.

### Screening nach dem TPS1-Gen von H.polymorpha

Um die DNA der einzelnen Kolonien analysieren zu können, wurden (gemäß Sambrook *et al.,* 1989) Nitrozellulose-Membranen sorgfältig auf die Platten gelegt. Mit einer dünnen Nadel wurden, asymmetrisch verteilt, vier Löcher durch Membran und Gel als Markierungen gestochen, um später die Orientierung der Membranen auf den Platten reproduzieren zu können. Durch das Abziehen der Membranen entstand eine Replikation der auf der Platte vorhandenen Kolonien.

Als nächstes wurden vier Plastikschalen mit 3MM Saugpapier (Whatman) ausgelegt und mit je einer von vier verschiedenen Lösungen benetzt. Überschüssige Flüssigkeit wurde verworfen. Die Nitrozellulose-Membranen wurden (mit den Kolonien nach oben) zuerst 3 min auf mit 10% (w/v) SDS getränktes Saugpapier gelegt. Dann wurden sie in die zweite Schale mit Denaturierungslösung (0,5 N NaOH, 1,5 M NaCl) gegeben und 5 min lang dort gehalten. Anschließend wurden sie nacheinander je 5 min auf Saugpapieren mit Neutralisierungslösung (1,5 M NaCl, 0,5M Tris-HCl, pH 7,4) bzw. 2 x SSC (10 x SSC 1,5 M NaCl, 170 mM Natriumcitrat) belassen. Um die DNA an die Nitrozellulose zu fixieren, wurden die Membranen zwischen je zwei 3MM Saugpapieren gelegt und 2 h lang bei 80°C in einem Vakuumofen gebacken. Als nächstes wurden die Membranen 5 min in 2 x SSC benetzt und dann 30 min bei 50°C in eine Vorwaschlösung (5 x SSC, 0,5% [w/v] SDS, 1 mM EDTA, pH 8.0) getaucht. Mit einem nassen Kleenex wurde überschüssiges, bakterielles Material weggewischt, bevor die Membranen 2 h bei 68°C in Vorhybridisierungslösung (6x SSC, 0,25% [w/v] Magermilch-pulver) gelegt wurden. Für die eigentliche Hybridisierung wurde die radioaktiv markierte *TPS1* Sonde mit insgesamt ca. 1x10⁷ cpm (siehe Abschnitt "Herstellung einer radioaktiven *TPS1* Sonde") in 40 ml Vorhybridisierunglösung gegeben und die Membranen darin über Nacht bei 68°C inkubiert. Nachdem die Membranen dann dreimal kurz in 2 x SSC, 0,1% (w/v) SDS gespült und anschließend während 1 h bei 68°C in 1 x SSC, 0,1% (w/v) SDS gewaschen worden waren, wurden sie getrocknet und auf BIOMAX-Film exponiert. Aufgrund der Signale auf den entwickelten Filmen konnten auf den Platten 8 positive Kolonien gepickt und davon Stocks angelegt werden. Aus diesen Kolonien wurden die Plasmide extrahiert. Mit PCR wurde getestet, ob das 650 bp Fragment wirklich vorhanden war.

### Beispiel 2

### Sequenzierung des TPS1-Gens von H. polymorpha

### Plasmidisolierung

Für die Sequenzierung wurden zwei Kolonien ausgewählt, die mittels PCR mit Primer von innerhalb des 650 bp Fragments nach aussen (F4 und R4, siehe Tabelle 1) und solchen vom Plasmid her Richtung Insert (Plasm. F und Plasm. R, siehe Tabelle 1) möglichst grosse Banden ergaben. Von diesen beiden Kolonien (Nr. 20.1 und 21.3) wurden mit dem Plasmid Midi Kit (Qiagen) reine Plasmidextrakte hergestellt.

### Sequenzierung

Sequenzen wurden mit einem zyklischen Sequenzierprogramm (PCR-Gerät: Progene) und dem Sequenzierautomaten ABI 301 (Perkin Elmer) hergestellt. Dazu wurden 0,5 µl (0,5 µg) Plasmid-DNA, 1 µl Primer (0,5 µM Endkonzentration), 4 µl Reaktionsmix (DNA Sequencing Kit) und 4 µl Wasser eingesetzt. Das benutzte Sequenzierprogramm beinhaltete 27 Zyklen à 30 s bei 96°C, 15 s bei 50°C, 4 min bei 60°C. Nach Ablauf des Programms wurde der Reaktion 10 µl Wasser hinzugefügt und die DNA mit Natriumacetat und Ethanol gefällt. Das Pellet wurde zweimal mit 1 ml eiskaltem 70% (v/v) Ethanol gewaschen. Die DNA wurde dann kurz getrocknet und in 25 µl TSR (template suppressing reagent, DNA Sequencing Kit) resuspendiert. Nach zweiminütiger Inkubation waren die Proben nun bereit für die Sequenzierung im ABI 301.

Die für die Sequenzierung des Plasmids aus dem Klon Nr. 21.3 verwendeten Primer sind in Tabelle 1 aufgelistet Sie wurden an "Expedite™ Nucleic Acid Synthesis"-Geräten am FMI hergestellt. Die Sequenzen wurden mittels GCG-Programm (Devereux *et al.,* 1984) analysiert.

**Tabelle 1**

| Liste der für die Sequenzierung des *TPS1-*Gens verwendeten Primer | | | | |
|---|---|---|---|---|
| **Name** | **Richtung** | **Länge (bp)** | **Sequenz** | |
| **F3** | vorwärts | 23 | 5' GGAAGCAAATAAACTGTTTTGCC 3' | (SEQ ID NO:11) |
| **F4** | vorwärts | 23 | 5' CTGTAAGTGCTTATCCGATTGGC 3' | (SEQ ID NO:12) |
| **F6** | vorwärts | 22 | 5' GGACGACAAACTGTCGAGCGGG 3' | (SEQ ID NO:13) |
| **F7** | vorwärts | 22 | 5' CATACTCCTTTTCCTTCAAGCG 3' | (SEQ ID NO:14) |
| **F8** | vorwärts | 21 | 5' AAAGCGTGAACTTCCAAGAGC 3' | (SEQ ID NO:15) |
| **F9** | vorwärts | 22 | 5' GCGTGTGATTACTGTGGTTTGC 3' | (SEQ ID NO:16) |
| **F10** | vorwärts | 26 | 5'GGTGAGATAATATTTTCGAAATTTCC 3' | (SEQ ID NO:17) |
| **F11** | vorwärts | 27 | 5'CCCATCAAATGCAGCAAGATATTGACC3' | (SEQ ID NO:18) |
| **R3** | rückwärts | 21 | 5' CCATTCAAGAATTTGTCAACG 3' | (SEQ ID NO:19) |
| **R4** | rückwärts | 23 | 5' CATGAGATGATAATCATGTACCC 3' | (SEQ ID NO:20) |
| **R5** | rückwärts | 23 | 5'CAATTTTGACATTCGGTAGCCCC 3' | (SEQ ID NO:21) |
| **R6** | rückwärts | 22 | 5' GTAATGCCGTCACTAATCCGCC 3' | (SEQ ID NO:22) |
| **R7** | rückwärts | 23 | 5' GAACATCTTCTGAAAATTGCCCC 3' | (SEQ ID NO:23) |
| **R8** | rückwärts | 21 | 5' CTAGCTCATTTACAGCTGCCC 3' | (SEQ ID NO:24) |
| **R9** | rückwärts | 25 | 5' CATAGCTTTCGAGCCTTTCATCTGG 3' | (SEQ ID NO:25) |
| **Plasm F** | vorwärts | 24 | 5' GGCGAGCCCGATCTTCCCCATCGG 3' | (SEQ ID NO:26) |
| **Plasm R** | rückwärts | 26 | 5'CTGCTCGCTTCGCTACTTGGAGCCAC3' | (SEQ ID NO:27) |

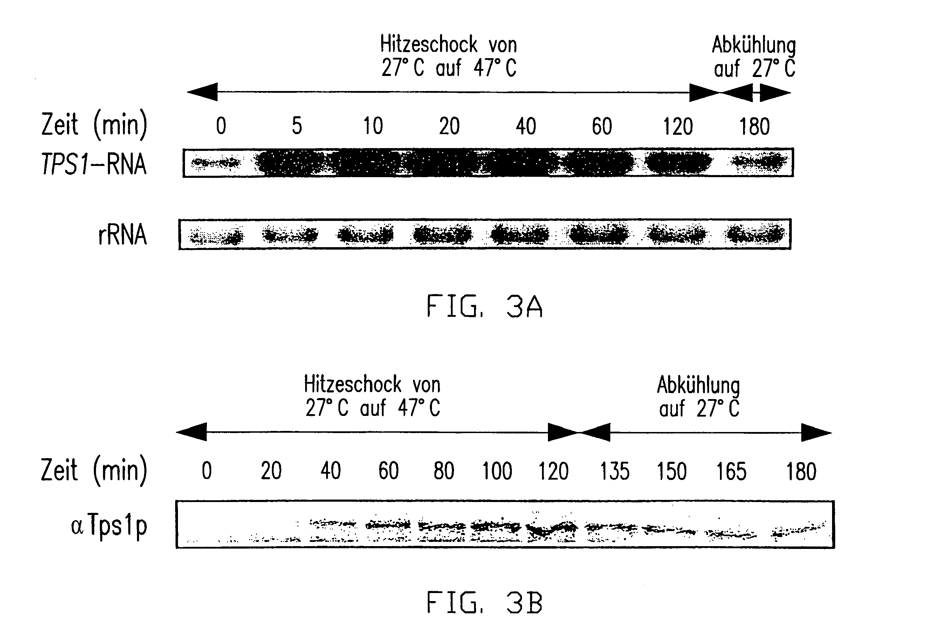

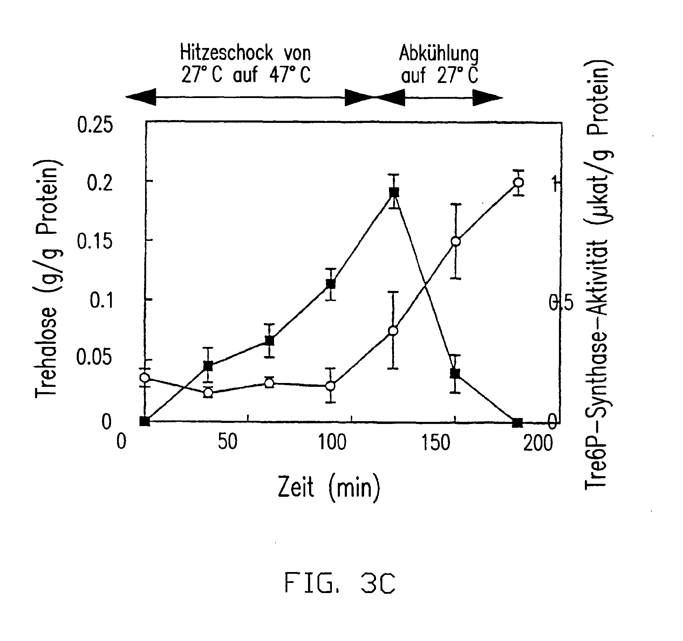

In der Folge wird nun ein aus *H. polymorpha* selbst isolierter Pomotor und seine Wirkungsweise genauer beschrieben. Dieser Promotor, der die Expression von TPS1 steuert, wurde untersucht, indem die Zunahme von *TPS1-*mRNA unter bestimmten Bedingungen gemessen wurde. Dabei wurde festgestellt, daß dieser Promotor zwar bei für *H. polymorpha* sehr tiefen Temperaturen bereits geringe Mengen TPS1 exprimiert, daß aber diese Expression bei hohen Temperaturen sehr stark zunimmt, d.h. weitaus stärker als bei bisher beschriebenen hitzeschockinduzierten Promotoren (siehe Figur 3A, Northern Blott des Hitzeschockes).Die hitzeinduzierte Zunahme der *TPS1-*mRNA korreliert mit der Zunahme des Tps1-Proteins (Figur 3B), mit der Zunahme der Trehalose-6-Phosphat Synthaseaktivität und mit der Zunahme der intrazellulären Trehalosekonzentration (Figur 3C). Um den Hitzeeinfluss zu optimieren kann der Promotor z.B. gezielt verkürzt und mit weiteren HSE enthaltenden Segmenten gekoppelt werden.

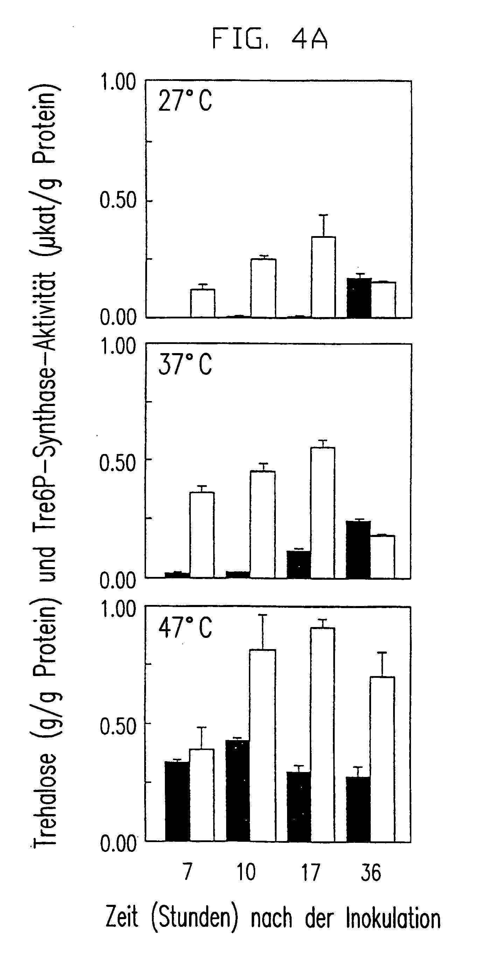

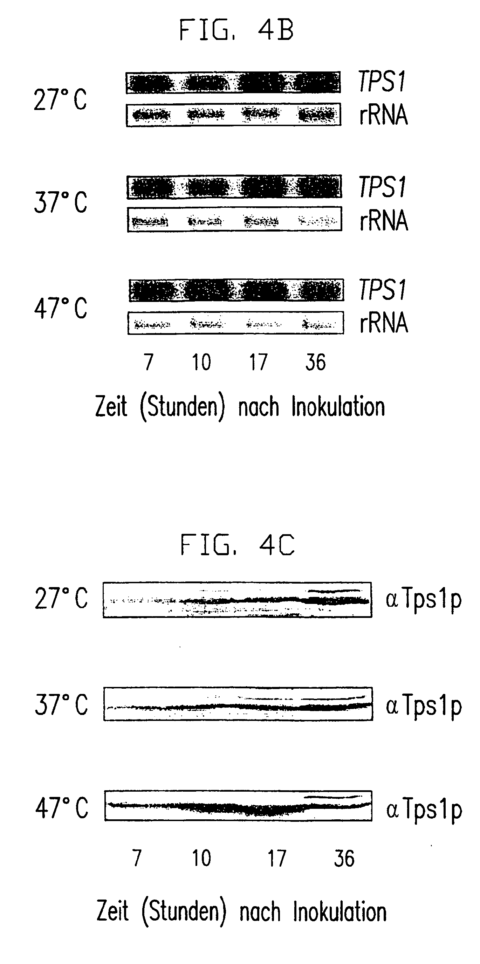

Neben der Hitze-Induktion wurde - wie aufgrund der engen biologischen Beziehung der beiden Stressfaktoren erwartet - auch eine vom Glukose-Entzug abhängige Trehaloseakkumulation gefunden (siehe Figur 4A). Diese Trehaloseakkumulation korreliert mit der Zunahme der Trehalose-6-Phosphat-Synthase-Aktivität, der Zunahme der *TPS1-*mRNA (Figur 4B), und der Trehaloseakkumulation mit der Zunahme von Tps1-Protein während Glukoseentzug (Figur 4C).

Die überaus hohen Akkumulationswerte von *TPS1* mRNA deuten auf eine hohe Stabilität der TPS1 mRNA hin, was diese (bzw. die darauf gründende cDNA oder daraus erhältliche Information) nicht nur zu einem wertvollen Hilfsmittel bei der Isolierung des Promotors macht sondern auch zu einem besonders wertvollen Mittel zum Schutz anderer Organismen vor verschiedenen Stressbedingungen, wie zum Beispiel Hitze oder Trockenheit. Mit geeigneten Promotoren und Vektoren (wie z.B. in WO 93/17093 und WO 96/00789 beschrieben) versehene *TPS1*-DNA kann beispielsweise zum Schutz von Pflanzen gegen Austrocknung eingesetzt werden, wodurch diese in wärmeren und niederschlagsärmeren Gebieten angepflanzt werden können. Selbstverständlich können zu diesem Zweck nicht nur *TPS1-*DNA selbst, sondern auch damit verwandte DNA eingesetzt werden.

### Beispiel 3

### Vergleichende Expression eines bakteriellen lacZ-Gens unter Kontrolle des FMD- und des TPS1-Promotors

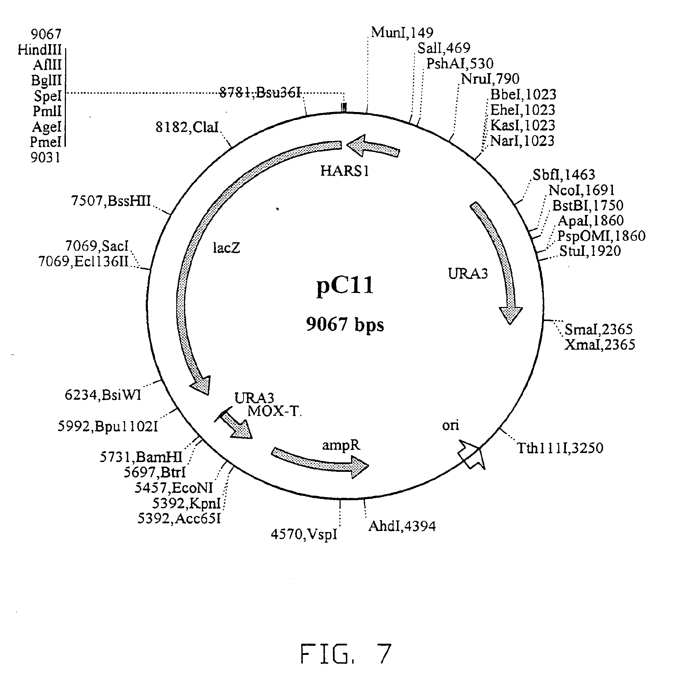

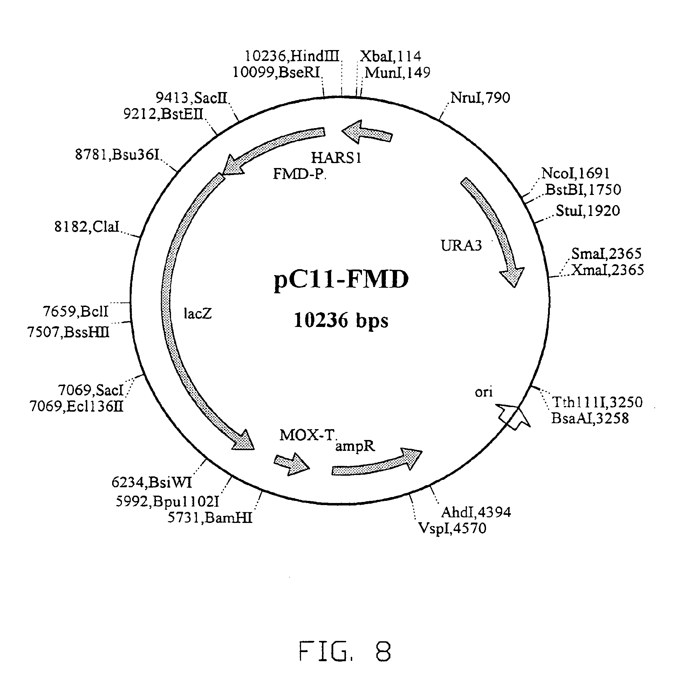

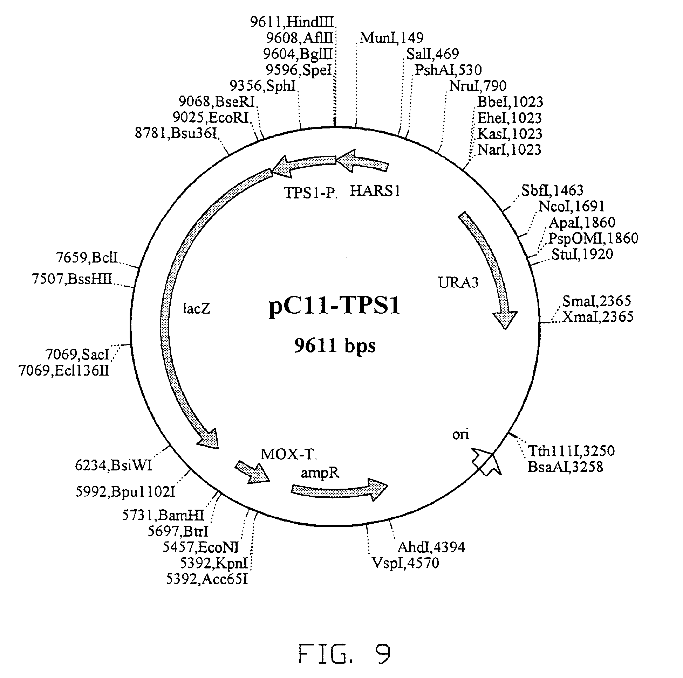

Ausgehend von dem integrativen *H. polymorpha-Vektor* pC11 (Figur 7) wurden zwei Derivate konstruiert, die sich nur durch den jeweiligen Promotor vor dem lacZ-Reportergen unterscheiden. Bei pC11-FMD (Figur 8) befindet sich das lacZ-Gen unter der Kontrolle des bereits gut charakterisierten *FMD-*Promotors, bei pC11-TPS1 (Figur 9) unter der Kontrolle des zu testenden hitzeinduzierbaren Promotors. Für dieses Experiment wurde als hitzeinduzierbare Promotor das Fragment der unter SEQ ID NO:1 angegebenen Sequenz zwischen den Nukleotiden 228 und 792 (im folgenden *TPS1-*Promotor genannt) eingesetzt.

*H. polymorpha* RB11 wurde mit pC11-FMD oder pC11-TPS1 transformiert (siehe Materialien und Methoden), und aus je etwa 1000 Uracil-prototrophen Zellklonen wurden separat stabile Stämme erzeugt, bei denen das jeweilige Plasmid genomisch stabil integriert vorlag. Dies geschah folgendermaßen: Nach erfolgte Transformation wurden die Ansätze auf Selektionsmediumplatten ausplattiert. Nach drei Tagen wurden makroskopische Einzelkolonien sichtbar. Je 1000 freistehende Einzelkolonien wurden steril auf neue Selektionsplatten übertragen, die dann für zwei Tage bei 37°C inkubiert wurden. Dieser Vorgang wurde zwei weitere Male wiederholt (Passagierung). Anschließend wurden die Zellklone auf Vollmediumplatten übertragen und erneut für zwei Tage bei 37°C inkubiert (Stabilisierung). Abschließend wurden die Zellklone erneut auf Selektionsplatten übertragen, wobei eventuelle noch frei vorliegende Plasmide verlorengehen sollen. Nach zweitägiger Inkubation dieser Platten bei 37°C war die Stammerzeugung abgeschlossen. Die genaue Kopienzahl sowie die Integrations-Loci der Plasmide in den einzelnen Stämmen wurden nicht bestimmt, nach Gatzke *et al.* (1995) sollten sich jedoch die verschiedenen erzeugten Stämme diesbezüglich deutlich voneinander unterscheiden

Da sowohl die Kopienzahl als auch die genomische Umgebung die Transkriptionsrate eines Gens stark beeinflussen, mußte angenommen werden, daß sich einzelne Zellklone auch hinsichtlich ihrer β-Galaktosidase-Aktivitäten stark voneinander unterscheiden. Dies konnte experimentell bestätigt werden (Daten nicht gezeigt). Es war daher nicht möglich, mit Hilfe von Einzelstämmen Promotorstärken direkt zu vergleichen. Um dennoch objektive Promotorstudien durchführen zu können, wurden je etwa 500 separat erzeugte Einzelstämme vereinigt, wobei hinsichtlich Kopienzahl und Integrations-Loci repräsentative Stammgemische entstehen sollten. Da die den Stammerzeugungungen zugrunde liegenden Plasmide pC11-FMD und pC11-TPS1 bis auf den jeweiligen, vor dem lacZ-Gen positionierten Promotor identisch sind, kann angenommen werden, daß sie in homologer Weise in das Wirtsgenom integrieren. Diese Annahmen wurde durch den Befund unterstützt, daß sich die β-Galaktosidase-Aktivitäten verschiedener Stammgemische derselben Transformation nur geringfügig voneinander unterschieden (Daten nicht gezeigt). Somit sollte die Bestimmung von β-Galaktosidase-Aktivitäten von Stammgemischen, die aus der Transformation mit weitgehend identischen Plasmiden hervorgegangen sind, in der Tat objektive Promotor-Vergleiche in *H. polymorpha* ermöglichen.

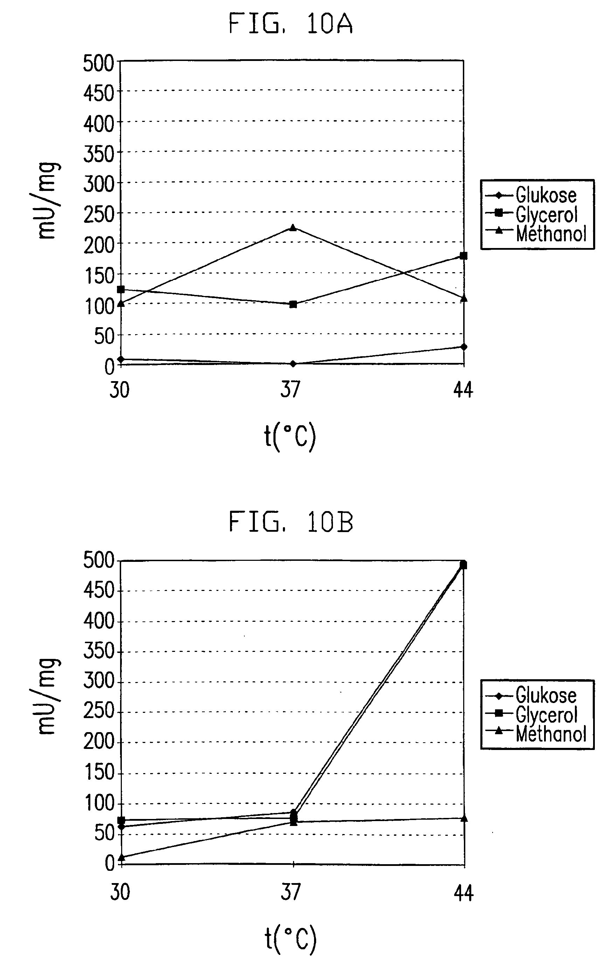

Die lacZ-Aktivitäten unter der Kontrolle von *FMD-* oder *TPS1-*Promotor wurden bei drei verschiedenen Temperaturen unter drei verschiedenen Kohlenstoffquellen durchgeführt (siehe Figur 10). Dazu wurden die oben beschriebenen Stammgemische bei den angegebenen Temperaturen und Kohlenstoffquellen in 10 ml Selektionsmedium bis zu einer OD₆₀₀ von 5 kultiviert und anschließend Zellextrakte hergestellt, deren β-Galaktosidase-Aktivitäten mittels ONPG-Flüssigmessungen bestimmt wurden. Dies geschah folgendermaßen: Nach Erreichen der gewünschten Dichte wurden die Kulturen 10 min lang bei 4°C zentrifugiert, die Zellpellets in 10 ml lacZ-Puffer (50 mM Natriumphosphat-Puffer, pH 7; 10 mM KCl; 1 mM MgSO₄) gewaschen, in 500 µl lacZ-Puffer resuspendiert und in 1,5 ml-Eppendorfgefäße überführt. Nach dem Versetzen der Suspensionen mit Glasperlen (Durchmesser 0,45 mm; bis zum Meniskus der Flüssigkeit) erfolgte der Zellaufschluß in einem Vibrax (Janke & Kunkel; 6 min; 4°C; 2200 rpm). Die Zell-Lysate wurden entnommen und 10 min lang zentrifugiert (Tischzentrifuge; 4°C; 10 min). Die löslichen Fraktionen wurden sowohl für die Bestimmung der β-Galaktosidase-Aktivitäten als auch für die Messung des Gesamtproteingehalts eingesetzt. Für die β-Galaktasidase-Aktivitätsmessungen wurden 1 ml ONPG-Lösung (4 mg ONPG/ml lacZ-Puffer) mit verschiedenen Verdünnungen der löslichen Fraktionen versetzt und in Plastikküvetten (1 cm) überführt. Anschließend wurde über einen Zeitraum von 3 min in 30 sec-Intervallen die OD₄₂₀ gemessen, um ΔE ermitteln zu können. Für die Bestimmung des Gesamtproteingehalts in den Zellextrakten wurden 790 µl H₂O mit je 10 µl der jeweiligen löslichen Fraktion gemischt (je nach Proteingehalt 1:10, 1:5, 1:2 verdünnt oder unverdünnt) und mit 200 µl Bradford-Reagenz (Biorad) versetzt. Nach 10min Inkubation bei Raumtemperatur wurde die OD₄₉₀ photometrisch bestimmt und gegen einen Kontrollansatz, der lacZ-Puffer anstelle von Zellextrakt enthielt, abgeglichen. Die Proteinkonzentration im Zellextrakt wurde dann mittels einer BSA-Eichkurve aus den Absorptionswerten ermittelt. Die Berechnung der spezifischen β-Galaktosidase-Aktivitäten erfolgte gemäß folgender Formel:

| | |
|---|---|
| Volumenaktivität (mU/mL) = ΔE V/ε d v Gesamtprotein | V: Gesamtvolumen |
| | v: Probenvolumen |
| | e: Extinktionskoeffizient (0,0045 mM cm) |
| | d: Schichtdicke (1 cm) |

Von FMD-Promotor ist bekannt, daß er hauptsächlich über die Art der Kohlenstoffquelle gesteuert wird, eine Temperaturabhängigkeit wurde bisher nicht beschrieben (EP-Patent Nr. 299108). Dies konnte durch die hier durchgeführten Messungen bestätigt werden (siehe Figur 10A). Die β-Galaktosidase-Aktivitäten zeigten unter Glukose-Bedingungen ein geringes Niveau (Glukose-Repression), während unter Glycerol- oder Methanol-Bedingungen weitaus höhere Werte gemessen wurden (Derepression bzw. Induktion). Temperaturänderungen führten keine dramatischen Änderungen in den Meßwerten herbei (siehe Figur 10A). Dies zeigte sich auch in dem hier eingesetzten Testsystem. Die β-Galaktosidase-Aktivitäten waren bei 30°C oder 37°C gering, stiegen jedoch bei 44°C drastisch an (siehe Figur 10B). Dieser temperaturabhängige Anstieg der Promotoraktivität erfolgte nicht unter Methanol-Bedingungen (Figur 10B), was bisher noch nicht beschrieben wurde. Überraschenderweise lagen die höchsten für *TPS1*-Promotor gemessenen β-Galaktosidase-Aktivitäten deutlich über denen für *FMD*-Promotor (siehe Figur 10A, B).

### Beispiel 4

### Vergleichende Expression eines Phytasegens unter Kontrolle des FMD- und des TPS1-Promotors

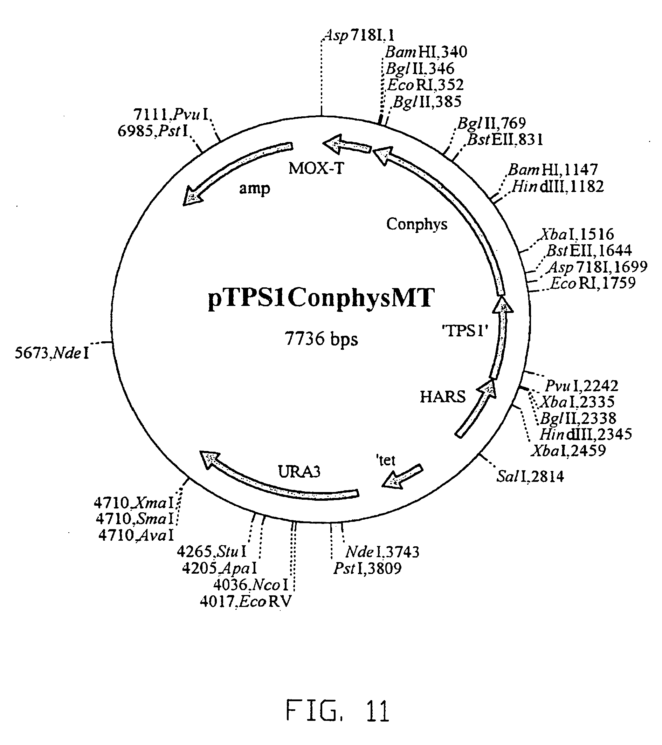

Rekombinante Stämme wurden durch Transformation mit den Vektoren pTPS1ConphysMT und pFMTConphysMT nach Standardverfahren generiert. Die beiden für die Transformation genutzten Vektoren sind mit der Ausnahme des Promotorelementes in der Expressionskassette identisch. pTPS1ConphysMT enthält als als hitzeinduzierbare Promotor das Fragment der unter SEQ ID NO:1 angegebenen Sequenz zwischen den Nukleotiden 228 und 792, dessen 3'-Ende eine *EcoRI*-Schnittstelle aufweist (im folgenden *TPS1*-Promotor genannt), pFMTConPhysMT den *FMD***-Promotor.** Plasmidkarte und Nukleotidsequenz des Vektors pTPS1ConphysMT sind in Figur 11 dargestellt. Als Reportergen dient ein Mutein einer Phytase.

Nach Transformation durch Elektroporation wurden rekombinante *H. polymorpha-*Stämme durch Wachstum der aus der Transformation hervorgegangenen Uracil-prototrophen Klone auf selektives Medium über mindestens 80 Generationen erhalten (Gatzke *et al.,* 1995). Repräsentative Transformanden der beiden entstandenen Stammkollektionen wurden vergleichend unter verschiedenen Bedingungen im 3 ml Maßstab kultiviert. Die Kultivierung erfolgte in einem mit 0,1 M Phosphatpuffer pH 5,0 gepufferten YNB-Medium supplementiert mit 2% Glukose oder 5% Glyzerin. Die Quantifizierung der sezernierten Phytase erfolgte nach 48 Stunden mit Hilfe der in den Materialien und Methoden beschriebenen Methode aus Aliquots der Kulturüberstände.

| Temperatur | FMD-Conphys | | | TPS1Conphys | | | |
|---|---|---|---|---|---|---|---|
| | mg/L | OD₆₀₀ | mg/OD | mg/L | OD₆₀₀ | mg/OD | |
| **37°C** | 2,185 | 1,453 | **1,500** | 2,026 | 1,104 | **1,840** | Glyzerin |
| | | | | 2,028 | 0,626 | **3,240** | Glukose |
| **40°C** | 0,916 | 0,618 | **1,480** | 1,336 | 0,697 | **1,920** | Glyzerin |
| | | | | 2,379 | 0,448 | **5,300** | Glukose |
| **44°C** | 0,706 | 0,774 | **0,910** | 1,219 | 0,671 | **1,820** | Glyzerin |
| | | | | 1,394 | 0,418 | **3,330** | Glukose |

In dieser Studie wurde der *TPS1*-Promotor mit dem stärksten bisher genutzten Promotor, dem *FMD*-Promotor verglichen. Die Nutzung des *TPS1*-Promotors führte dabei zu leicht erhöhten Expressionswerten bei 37°C gegenüber dem *FMD*-Promotor. Bei 40°C und 44°C wurde bei Nutzung des *TPS1*-Promotors eine 2-3fach höhere Expression gegenüber dem *FMD*-Promotor beobachtet.

### Literaturverzeichnis

Bradford, M.M. (1976) A rapid and sensitive method for the quantitation of microgram quan-tities of protein utilizing the principle of protein-dye binding. Anal Biochem 72: 248-254.
Devereux, J., Haeberli, P. and Smithies, O. (1984) A comparative set of sequence analysis programs for the VAX. Nucl Acids Res 12: 387-395.
De Virgilio, C., Bürckert, N., Boller, T. and Wiemken, A. (1991) A method to study the rapid phosphorylation-related modulation of neutral trehalase activity by temperature shifts in yeast. FEBS Lett 291: 355-358.
Faber, K.N., Swaving, G.J., Faber, F., Ab, G., Harder, W., Veenhuis, M. and Haima, P. (1992) Chromosomal targeting of replicating plasmids in the yeast Hansenula polymorpha. J Gen Microbiol 138: 2405-2416.
Gatzke, R., Weydemann, U., Janowicz, Z. A. & Hollenberg, C. P. (1995) Stable multicopy integration of vector sequences in Hansenula polymorpha. Appl. Microbiol. Biotechnol 43, 844-849.)
Hottiger, T., Schmutz, P. and Wiemken, A. (1987) Heat-induced accumulation and futile cycling of trehalose in Saccharomyces cerevisiae. J Bacteriol 169: 5518-5522.
Huxley, C., Green, E.D. and Dunham I. (1990) Rapid assessment of Saccharomyces cerevisiae mating type by PCR. Trends Genet 6 (8): p. 236.
Laemmli, U. K. (1970) Cleavage od structural proteins during the assembly of the head of bacteriophage T4. Nature 227:680-685.
Levine, D.W. and Cooney, C.L. (1973) Isolation and characterization of a thermotolerant methanol-utilizing yeast. Appl Microbiol 26: 982-990.
Parrou, J.L. and François, J. (1997) A simplified procedure for a rapid and reliable assay of both glycogen and trehalose in whole yeast cells. Anal Biochem 248: 186-188.
Peterson, G.C. (1977) A simplification of the protein assay method of Lowry et al. which is more generally applicable. Anal Biochem 83: 346-356.
Piper, P.W. (1994) Measurement of transcription. In: MolecularGenetics of yeast. A practical approach, J.R. Johnston (Hrsg.). IRL Press, Oxford.
Sambrook, J., Fritsch, E.F. and Maniatis, T. (1989) Molecular Cloning: A Laboratory Manual. Second edition. Cold Spring Harbor Laboratory Press, Cold Spring Harbor.
Weydemann U, Keup P, Piontek M, Strasser AWM, Schweden J, Gellissen G, Janowicz ZA (1995) High-level secretion of hirudin by Hansenula polymorpha - authentic processing of three different preprohirudins. Appl Microbiol Biotechnol 44:844-849

## Patentansprüche

1. Nukleinsäure-Molekül, das einen hitzeinduzierbaren Promotor umfasst, kein STRE-Element mit der Sequenz CCCCT oder AGGGG enthält und aus folgenden Nukleinsäuren ausgewählt ist:
(a) einer Nukleinsäure mit der in SEQ ID NO: 1 angegebenen Sequenz;
(b) einer Nukleinsäure mit einer Sequenz, die auf einer Länge von 300 bp mindestens 60 % Identität mit der in (a) angegebenen Sequenz aufweist, wobei die Sequenzidentität mit einem Lücken-Wert von 50 und einem Lückenlängen-Wert von 3 bestimmt wird;
(c) einer Nukleinsäure, die mit dem Gegenstrang einer der in (a) oder (b) angegebenen Nukleinsäuren unter stringenten Bedingungen hybridisiert;
(d) einem Fragment einer der in (a) bis (c) angegebenen Nukleinsäuren, das die Funktion des hitzeinduzierbaren Promotors behält;
(e) einer Kombination mehrerer der in (a) bis (d) angegebenen Nukleinsäuren, wobei die Sequenzen der Nukleinsäuren gleich oder verschieden sein können;
oder
ein Nukleinsäure-Molekül mit einer Sequenz, die zu der Sequenz einer der in (a) bis (e) angegebenen Nukleinsäuren komplementär ist.

2. Nukleinsäure-Molekül gemäß Anspruch 1, **dadurch gekennzeichnet, dass** die unter (b) angegebene Nukleinsäure mindestens 80% Identität mit der in (a) angegebenen Sequenz aufweist, oder ihre Komplementärsequenz.

3. Nukleinsäure-Molekül gemäß Anspruch 1, **dadurch gekennzeichnet, dass** die unter (b) angegebene Nukleinsäure mindestens 90% Identität mit der in (a) angegebenen Sequenz aufweist, oder ihre Komplementärsequenz.

4. Nukleinsäure-Molekül gemäß Anspruch 1, **dadurch gekennzeichnet, dass** die unter (b) angegebene Nukleinsäure mindestens 95% Identität mit der in (a) angegebenen Sequenz aufweist, oder ihre Komplementärsequenz.

5. Nukleinsäure-Molekül gemäß einem der vorangegangenen Ansprüche, **dadurch gekennzeichnet, dass** es mindestens ein Hitzeschock-Element mit der Sequenz NGAANNNNNNNGAAN (SEQ ID NO: 2) oder deren Komplementärsequenz aufweist, wobei die mit N bezeichneten Nukleotide unabhängig voneinander A, T, C und G sein können.

6. Nukleinsäure-Molekül gemäß Anspruch 5, **dadurch gekennzeichnet, dass** es mindestens ein Hitzeschock-Element mit der Sequenz NGAANNBWMNNGAAN (SEQ ID NO: 3) oder deren Komplementärsequenz aufweist, wobei B ein G, C oder T, W ein A oder T, und M ein C oder A ist.

7. Nukleinsäure-Molekül gemäß Anspruch 6, **dadurch gekennzeichnet, dass** das Hitzeschock-Element aus TGAAGCCTCTTGAAA (SEQ ID NO: 4) und/oder TGAATATAAAGGAAA (SEQ ID NO: 5) und/oder deren Komplementär-sequenzen ausgewählt sind, wobei wenn zwei oder mehrere Hitzeschock-Elemente vorhanden sind, diese gleiche oder verschiedene Sequenzen aufweisen können.

8. Nukleinsäure-Molekül gemäß Anspruch 5, 6 und 7, **dadurch gekennzeichnet, dass** es mindestens zwei verschiedene Hitzeschock-Elemente aufweist.

9. Nukleinsäure-Molekül gemäß Anspruch 1, **dadurch gekennzeichnet, dass** das unter (d) angegebene Fragment die Sequenz von Nukleotid 228 bis Nukleotid 792 in der SEQ ID NO: 1 umfasst.

10. Nukleinsäure-Molekül gemäß Anspruch 1, **dadurch gekennzeichnet, dass** das unter (d) angegebene Fragment die Sequenz von Nukleotid 492 bis Nukleotid 792 in der SEQ ID NO: 1 umfasst.

11. Nukleinsäure-Molekül gemäß Anspruch 1, **dadurch gekennzeichnet, dass** das unter (d) angegebene Fragment die Sequenz von Nukleotid 627 bis Nukleotid 713 in der SEQ ID NO: 1 umfasst.

12. Nukleinsäure-Molekül gemäß einem der vorangegangenen Ansprüche, **dadurch gekennzeichnet, dass** es weiterhin mindestens eine unter der Transkriptionskontrolle des hitzeinduzierbaren Promotors stehende Nukleinsäuresequenz für ein heterologes Gen umfasst.

13. Nukleinsäure-Molekül gemäß einem der Ansprüche 1 bis 11, **dadurch gekennzeichnet, dass** es weiterhin eine unter der Transkriptionskontrolle des hitzeinduzierbaren Promotors stehende Nukleinsäuresequenz umfasst, die aus folgenden Sequenzen ausgewählt wird:
(i) einer Nukleinsäuresequenz, die ein Polypeptid mit der Aminosäure-sequenz der Trehalose-6-Phosphat-Synthase von Hansenula polymorpha kodiert;
(ii) einer Nukleinsäuresequenz wie in SEQ ID NO: 6 angegeben;
(iii) einer Nukleinsäuresequenz, die mindestens 80% Identität mit der in SEQ ID NO: 6 angegebenen Sequenz aufweist;
(iv) einer Nukleinsäuresequenz, die ein Polypeptid mit der in SEQ ID NO: 7 angegebenen Aminosäuresequenz oder mit einer Teilsequenz davon kodiert, wobei das Polypeptid Trehalose-6-Phosphat-Synthase-Aktivität aufweist;
(v) einer Nukleinsäuresequenz, die unter Berücksichtigung der Entartung des genetischen Codes ein Polypeptid mit der in SEQ ID NO: 7 angegebenen Aminosäuresequenz oder mit einer Teilsequenz davon kodieren würde, wobei das Polypeptid Trehalose-6-Phosphat-Synthase-Aktivität aufweist;
(vi) einer Nukleinsäuresequenz, die ein Polypeptid kodiert, dessen Aminosäuresequenz mindestens 80% identisch mit der in SEQ ID NO: 7 angegebenen Aminosäuresequenz ist.

14. Nukleinsäure-Molekül nach Anspruch 13, **dadurch gekennzeichnet, dass** die unter (iii) angegebene Nukleinsäuresequenz mindestens 90%Identität mit der in SEQ ID NO: 6 angegebenen Sequenz aufweist.

15. Nukleinsäure-Molekül nach Anspruch 13, **dadurch gekennzeichnet, dass** die unter (vi) angegebene Nukleinsäuresequenz ein Polypeptid kodiert, dessen Aminosäuresequenz mindestens 90% identisch mit der in SEQ ID NO: 7 angegebenen Aminosäuresequenz ist.

16. Wirtszelle, enthaltend einen Expressionsvektor, der mindestens ein Nukleinsäure-Molekül nach einem der Ansprüche 1 bis 11 umfasst, wobei die Wirtszelle eine prokaryontische oder eukaryontische Zelle ist.

17. Wirtszelle gemäß Anspruch 16, **dadurch gekennzeichnet, dass** die eukaryontische Zelle eine Pilzzelle ist.

18. Wirtszelle gemäß Anspruch 17, **dadurch gekennzeichnet, dass** die Pilzzelle eine Hefezelle ist.

19. Wirtszelle gemäß Anspruch 18, **dadurch gekennzeichnet, dass** die Hefezelle *Hansenula polymorpha* ist.

20. Expressionsvektor, der mindestens ein Nukleinsäure-Molekül nach einem der Ansprüche 1 bis 11 umfasst.

21. Expressionsvektor, der mindestens ein Nukleinsäure-Molekül nach einem der Ansprüche 12 bis 15 umfasst.

22. Kit, umfassend:
(a) einen Expressionsvektor nach Anspruch 20, der zum Hineinklonieren einer Nukleinsäure geeignet ist, welche ein rekombinantes Protein kodiert, und
(b) eine zur Induktion des hitzeinduzierbaren Promotors und zum Herstellen des rekombinanten Proteins geeignete Wirtszelle.

23. Kit, umfassend:
(a) einen Expressionsvektor nach Anspruch 21 und
(b) eine Wirtszelle, die zur Induktion des hitzeinduzierbaren Promotors und zur Herstellung eines von einer kodierenden Sequenz unter der Transkriptionskontrolle des hitzeinduzierbaren Promotors kodierten Proteins geeignet ist.

24. Verwendung eines Nukleinsäure-Moleküls nach einem der Ansprüche 1 bis 15 oder einer Wirtszelle nach einem der Ansprüche 16 bis 19 oder eines Expressionsvektors nach Anspruch 20 oder 21 oder eines Kits nach Anspruch 22 oder 23 zur Expression eines Gens unter der Kontrolle des hitzeinduzierbaren Promotors.

25. Verwendung eines Nukleinsäure-Moleküls nach einem der Ansprüche 1 bis 15 oder einer Wirtszelle nach einem der Ansprüche 16 bis 19 oder eines Expressionsvektors nach Anspruch 20 oder 21 oder eines Kits nach Anspruch 22 oder 23 zum Herstellen von einem oder mehreren Proteinen.

26. Verfahren zum Herstellen von einem oder mehreren Proteinen, umfassend:
(i) Hineinklonieren von mindestens einer Nukleinsäure, die ein rekombinantes Protein kodiert, in einen Expressionsvektor gemäss Anspruch 20, sodass die hineinklonierte Nukleinsäure unter der Transkriptionskontrolle des hitzeinduzierbaren Promotor steht;
(ii) Einführen des in (i) erhaltenen Expressionsvektors in eine zur Induktion des hitzeinduzierbaren Promotors und zum Herstellen des rekombinanten Proteins geeignete Wirtszelle;
(iii) Kultivieren der in (ii) erhaltenen Wirtszelle;
(iv) Induzieren des hitzeinduzierbaren Promotors in an sich bekannter Weise.

27. Verfahren zum Herstellen von einem oder mehreren Proteinen, umfassend:
(i) Einführen eines Expressionsvektors gemäß Anspruch 21 in eine zur Induktion des hitzeinduzierbaren Promotors und zum Herstellen des rekombinanten Proteins geeignete Wirtszelle;
(ii) Kultivieren der in (i) erhaltenen Wirtszelle;
(iii) Induzieren des hitzeinduzierbaren Promotors in an sich bekannter Weise.

## Claims

1. Nucleic acid molecule which comprises a heat-inducible promoter, contains no STRE element with the sequence CCCCT or AGGGG and is chosen from the following nucleic acids:
(a) a nucleic acid with the sequence shown in SEQ ID NO: 1;
(b) a nucleic acid with a sequence which over a length of 300 bp has at least 60 % identity with the sequence mentioned in (a), the sequence identity being determined with a gap value of 50 and a gap length value of 3;
(c) a nucleic acid which hybridizes under stringent conditions with the counter-strand of one of the nucleic acids mentioned in (a) or (b);
(d) a fragment of one of the nucleic acids mentioned in (a) to (c) which retains the function of the heat-inducible promoter;
(e) a combination of several of the nucleic acids mentioned in (a) to (d), it being possible for the sequences of the nucleic acids to be identical or different;
or
a nucleic acid molecule with a sequence which is complementary to the sequence of one of the nucleic acids mentioned in (a) to (e).

2. Nucleic acid molecule according to claim 1, **characterized in that** the nucleic acid mentioned under (b) has at least 80 % identity with the sequence mentioned in (a), or its complementary sequence.

3. Nucleic acid molecule according to claim 1, **characterized in that** the nucleic acid mentioned under (b) has at least 90 % identity with the sequence mentioned in (a), or its complementary sequence.

4. Nucleic acid molecule according to claim 1, **characterized in that** the nucleic acid mentioned under (b) has at least 95 % identity with the sequence mentioned in (a), or its complementary sequence.

5. Nucleic acid molecule according to one of the preceding claims, **characterized in that** it contains at least one heat shock element with the sequence NGAANNNNNNNGAAN (SEQ ID NO: 2) or the complementary sequence thereof, wherein the nucleotides designated by N independently of each other can be A, T, C and G.

6. Nucleic acid molecule according to claim 5, **characterized in that** it contains at least one heat shock element with the sequence NGAANNBWMNNGAAN (SEQ ID NO: 3) or the complementary sequence thereof, wherein B is a G, C or T, W is an A or T, and M is a C or A.

7. Nucleic acid molecule according to claim 6, **characterized in that** the heat shock element is chosen from TGAAGCCTCTTGAAA (SEQ ID NO: 4) and/or TGAATATAAAGGAAA (SEQ ID NO: 5) and/or complementary sequences thereof, wherein if two or more heat shock elements are present, these can have identical or different sequences.

8. Nucleic acid molecule according to claim 5, 6 and 7, **characterized in that** it contains at least two different heat shock elements.

9. Nucleic acid molecule according to claim 1, **characterized in that** the fragment mentioned under (d) comprises the sequence from nucleotide 228 to nucleotide 792 in SEQ ID NO: 1.

10. Nucleic acid molecule according to claim 1, **characterized in that** the fragment mentioned under (d) comprises the sequence from nucleotide 492 to nucleotide 792 in SEQ ID NO: 1.

11. Nucleic acid molecule according to claim 1, **characterized in that** the fragment mentioned under (d) comprises the sequence from nucleotide 627 to nucleotide 713 in SEQ ID NO: 1.

12. Nucleic acid molecule according to one of the preceding claims, **characterized in that** it furthermore comprises at least one nucleic acid sequence for a heterologous gene which is under the transcription control of the heat-inducible promoter.

13. Nucleic acid molecule according to one of claims 1 to 11, **characterized in that** it furthermore comprises a nucleic acid sequence which is under the transcription control of the heat-inducible promoter and is chosen from the following sequences:
(i) a nucleic acid sequence which codes a polypeptide with the amino acid sequence of the trehalose 6-phosphate synthase of Hansenula polymorpha;
(ii) a nucleic acid sequence as shown in SEQ ID NO: 6;
(iii) a nucleic acid sequence which has at least 80 % identity with the sequence shown in SEQ ID NO: 6;
(iv) a nucleic acid sequence which codes a polypeptide with the amino acid sequence shown in SEQ ID NO: 7 or with a part sequence thereof, the polypeptide having trehalose 6-phosphate synthase activity;
(v) a nucleic acid sequence which, taking into account degeneration of the genetic code, would code a polypeptide with the amino acid sequence shown in SEQ ID NO: 7 or with a part sequence thereof, the polypeptide having trehalose 6-phosphate synthase activity;
(vi) a nucleic acid sequence which codes a polypeptide, the amino acid sequence of which is at least 80 % identical to the amino acid sequence shown in SEQ ID NO: 7.

14. Nucleic acid molecule according to claim 13, **characterized in that** the nucleic acid sequence mentioned under (iii) has at least 90 % identity with the sequence shown in (SEQ ID NO: 6.

15. Nucleic acid molecule according to claim 13, **characterized in that** the nucleic acid sequence mentioned under (vi) codes a polypeptide, the amino acid sequence of which is at least 90 % identical to the amino acid sequence shown in SEQ ID NO: 7.

16. Host cell containing an expression vector which comprises at least one nucleic acid molecule according to one of claims 1 to 11, the host cell being a prokaryotic or eukaryotic cell.

17. Host cell according to claim 16, **characterized in that** the eukaryotic cell is a fungus cell.

18. Host cell according to claim 17, **characterized in that** the fungus cell is a yeast cell.

19. Host cell according to claim 18, **characterized in that** the yeast cell is *Hansenula polymorpha.*

20. Expression vector which comprises at least one nucleic acid molecule according to one of claims 1 to 11.

21. Expression vector which comprises at least one nucleic acid molecule according to one of claims 12 to 15.

22. Kit comprising:
(a) an expression vector according to claim 20, which is suitable for cloning-in a nucleic acid which codes a recombinant protein, and
(b) a host cell suitable for induction of the heat-inducible promoter and for production of the recombinant protein.

23. Kit comprising:
(a) an expression vector according to claim 21 and
(b) a host cell which is suitable for induction of the heat-inducible promoter and for production of a protein coded by a coding sequence under the transcription control of the heat-inducible promoter.

24. Use of a nucleic acid molecule according to one of claims 1 to 15 or of a host cell according to one of claims 16 to 19 or of an expression vector according to claim 20 or 21 or of a kit according to claim 22 or 23 for expression of a gene under the control of the heat-inducible promoter.

25. Use of a nucleic acid molecule according to one of claims 1 to 15 or of a host cell according to one of claims 16 to 19 or of an expression vector according to claim 20 or 21 or of a kit according to claim 22 or 23 for production of one or more proteins.

26. Process for the production of one or more proteins, comprising:
(i) cloning in of at least one nucleic acid which codes a recombinant protein into an expression vector according to claim 20, so that the nucleic acid cloned in is under the transcription control of the heat-inducible promoter;
(ii) introduction of the expression vector obtained in (i) into a host cell suitable for induction of the heat-inducible promoter and for production of the recombinant protein;
(iii) culturing of the host cell obtained in (ii);
(iv) induction of the heat-inducible promoter in a manner known per se.

27. Process for the production of one or more proteins, comprising:
(i) introduction of an expression vector according to claim 21 into a host cell suitable for induction of the heat-inducible promoter and for production of the recombinant protein;
(ii) culturing of the host cell obtained in (i);
(iii) induction of the heat-inducible promoter in a manner known per se.

## Revendications

1. Molécule d'acide nucléique, qui comprend un promoteur thermo-inductible,
qui ne contient pas d'élément STRE ayant la séquence CCCCT ou AGGGG et qui est choisie parmi les acides nucléiques suivants :
(a) un acide nucléique avec la séquence indiquée dans SEQ ID NO : 1;
(b) un acide nucléique avec une séquence qui sur une longueur de 300 paires de bases présente une identité d'au moins 60 % avec la séquence indiquée dans (a), l'identité séquentielle étant déterminée avec une valeur interstitielle de 50 et une valeur de longueur interstitielle de 3 ;
(c) un acide nucléique qui hybridise sous des conditions strictes (DE= stringent) avec le brin opposé d'un acide nucléique indiqué dans (a) ou (b) ;
(d) un fragment d'un acide nucléique indiqué dans (a) à (c) qui garde la fonction du promoteur thermo-inductible ;
(e) une combinaison de plusieurs acides nucléiques indiqués dans (a) à (d), les séquences des acides nucléiques pouvant être identiques ou différentes ;
ou
une molécule d'acide nucléique avec une séquence, qui est complémentaire avec la séquence d'un des acides nucléiques indiqués dans (a) à (e).

2. Molécule d'acide nucléique selon la revendication 1, **caractérisée en ce que** l'acide nucléique indiqué sous (b) a au moins 80 % d'identité avec la séquence indiquée dans (a), ou sa séquence complémentaire.

3. Molécule d'acide nucléique selon la revendication 1, **caractérisée en ce que** l'acide nucléique indiqué sous (b) a au moins 90 % d'identité avec la séquence indiquée dans (a) ou sa séquence complémentaire.

4. Molécule d'acide nucléique selon la revendication 1, **caractérisée en ce que** l'acide nucléique indiqué sous (b) a au moins 95 % d'identité avec la séquence indiquée sous (a) ou sa séquence complémentaire.

5. Molécule d'acide nucléique selon l'une des revendications précédentes, **caractérisée en ce qu'**elle a au moins un élément de choc thermique ayant la séquence NGAANNNNNNNGAAN (SEQ ID NO : 2) ou sa séquence complémentaire, les nucléotides définis par N peuvent indépendamment l'un de l'autre être A, T, C et G.

6. Molécule d'acide nucléique selon la revendication 5, **caractérisée en ce qu'**elle a au moins un élément de choc thermique ayant la séquence NGAANNBWMNNGAAN (SEQ ID NO: 3) ou sa séquence complémentaire, B étant un G, C ou T, W un A ou un T, et M un C ou un A.

7. Molécule d'acide nucléique selon la revendication 6, **caractérisée en ce que** l'élément de choc thermique est choisi parmi TGAAGCCTCTTGAAA (SEQ ID NO: 4) et/ou TGAATATAAAGGAAA (SEQ ID NO: 5) et/ou leurs séquences supplémentaires, dans quel cas, lorsqu'il y a deux ou plusieurs éléments de choc thermique, ceux-ci peuvent présenter des séquences identiques ou différentes.

8. Molécule d'acide nucléique selon la revendication 5, 6 et 7, **caractérisée en ce qu'**elle présente au moins deux éléments de choc thermique différents.

9. Molécule d'acide nucléique selon la revendication 1, **caractérisée en ce que** le fragment indiqué sous (d) comprend la séquence du nucléotide 228 au nucléotide 792 dans la SEQ ID NO : 1.

10. Molécule d'acide nucléique selon la revendication 1, **caractérisée en ce que** le fragment indiqué sous (d) comprend la séquence du nucléotide 492 au nucléotide 792 dans la SEQ ID NO : 1.

11. Molécule d'acide nucléique selon la revendication 1, **caractérisée en ce que** le fragment indiqué sous (d) comprend la séquence du nucléotide 627 au nucléotide 713 dans la SEQ ID NO : 1.

12. Molécule d'acide nucléique selon l'une des revendications précédentes, **caractérisée en ce qu'**elle comprend en outre au moins une séquence d'acide nucléique pour un gène hétérologue qui se trouve sous le contrôle de transcription du promoteur thermo-inductible.

13. Molécule d'acide nucléique selon l'une des revendications 1 à 11, **caractérisée en ce qu'**elle comprend en outre une séquence d'acide nucléique se trouvant sous le contrôle de transcription du promoteur thermo-inductible, la séquence étant choisie parmi les séquences suivantes :
(i) une séquence d'acide nucléique qui encode un polypeptide avec la séquence d'acide aminé de la tréhalose-6-phosphate-synthase de Hansenula polymorpha ;
(ii) une séquence d'acide nucléique comme indiquée dans SEQ ID NO:6;
(iii) une séquence d'acide nucléique, qui a au moins 80 % d'identité avec la séquence indiquée dans SEQ ID NO : 6 ;
(iv) une séquence d'acide nucléique, qui encode un polypeptide avec la séquence d'acide aminé indiquée dans SEQ ID NO :7 ou avec une séquence partielle de celle-ci, le polypeptide présentant une activité de tréhalose-6-phosphate-synthase ;
(v) une séquence d'acide nucléique, qui, en tenant compte de la dégénérescence du code génétique, encoderait un polypeptide avec la séquence d'acide aminé indiquée dans SEQ ID NO : 7 ou avec une séquence partielle de celle-ci, le polypeptide présentant de l'activité de tréhalose-6-phosphate-synthase ;
(vi) une séquence d'acide nucléique, qui encode un polypeptide, dont la séquence d'acide aminé est au moins identique avec 80 % de la séquence d'acide aminé indiquée dans SEQ ID NO : 7.

14. Molécule d'acide nucléique selon la revendication 13, **caractérisée en ce que** la séquence d'acide nucléique indiquée sous (iii) est au moins identique avec 90 % de la séquence indiquée sous SEQ ID NO : 6.

15. Molécule d'acide nucléique selon la revendication 13, **caractérisée en ce que** la séquence d'acide nucléique indiquée sous (vi) encode un polypeptide, dont la séquence d'acide aminé est au moins identique avec 90% de la séquence d'acide aminé indiquée dans SEQ ID NO : 7.

16. Cellule hôte comprenant un vecteur d'expression, qui comprend au moins une molécule d'acide nucléique selon l'une des revendications 1 à 11, la cellule hôte étant une cellule procaryote ou eucaryote.

17. Cellule hôte selon la revendication 16, **caractérisée en ce que** la cellule eucaryote est une cellule de fungi.

18. Cellule hôte selon la revendication 17, **caractérisée en ce que** la cellule de fungi est une cellule de levure.

19. Cellule hôte selon la revendication 18, **caractérisée en ce que** la cellule de levure est *Hansenula polymorpha.*

20. Vecteur d'expression, qui comprend au moins une molécule d'acide nucléique selon l'une des revendications 1 à 11.

21. Vecteur d'expression qui comprend au moins une molécule d'acide nucléique selon l'une des revendications 12 à 15.

22. Kit, comprenant :
(a) un vecteur d'expression selon la revendication 20 qui est approprié à introduire par clonage (« hineinklonieren ») un acide nucléique, qui encode une protéine recombinante, et
(b) une cellule hôte appropriée à l'induction du promoteur thermo-inductible et à la fabrication de la protéine recombinant.

23. Kit comprenant :
(a) un vecteur d'expression selon la revendication 21 et
(b) une cellule hôte qui est appropriée à l'induction du promoteur thermo-inductible et à la fabrication d'une protéine encodée par une séquence encodant sous le contrôle de transcription du promoteur thermo-inductible.

24. Utilisation d'une molécule d'acide nucléique selon l'une des revendications 1 à 15 ou d'une cellule hôte selon l'une des revendications 16 à 19 ou d'un vecteur d'expression selon la revendication 20 ou 21 ou d'un kit selon la revendication 22 ou 23 pour l'expression d'un gène sous le contrôle du promoteur thermo-inductible.

25. Utilisation d'une molécule d'acide nucléique selon l'une des revendications 1 à 15 ou d'une cellule hôte selon l'une des revendications 16 à 19 ou d'un vecteur d'expression selon la revendication 20 ou 21 ou d'un kit selon la revendication 22 ou 23 pour fabriquer une ou une pluralité de protéines.

26. Procédé destiné à la fabrication d'une ou d'une pluralité de protéines, comprenant :
(i) Introduction par clonage d'au moins un acide nucléique qui encode une protéine recombinante, dans un vecteur d'expression selon la revendication 20, de sorte que l'acide nucléique introduite par clonage se trouve sous le contrôle du promoteur thermo-inductible ;
(ii) introduction du vecteur d'expression obtenu dans (i) dans une cellule hôte appropriée à l'induction du promoteur thermo-inductible et à la fabrication de la protéine recombinante ;
(iii) culture de la cellule hôte obtenue dans (ii) ;
(iv) induction du promoteur thermo-inductible de manière connue en soi.

27. Procédé destiné à la fabrication d'une ou d'une pluralité de protéines, comprenant :
(i) introduction d'un vecteur d'expression selon la revendication 21 dans une cellule hôte appropriée à l'induction du promoteur thermo-inductible et à la fabrication de la protéine recombinante ;
(ii) culture de la cellule hôte obtenue dans (i) ;
(iii) induction du promoteur thermo-inductible de manière connue en soi.
